# EUROPEAN PATENT SPECIFICATION

(11) **EP 1 976 509 B1**
(45) Date of publication and mention of the grant of the patent: **17.12.2014**
(21) Application number: 07804876.6
(22) Date of filing: 18.01.2007
(51) Int. Cl.: A61K 31/357, C07D 319/06

(54) **PPAR MODULATORS**
PPAR-MODULATOREN
MODULATEURS DES RÉCEPTEURS PPAR

(30) Priority: 18.01.2006 US 760241 P
(43) Date of publication of application: 08.10.2008
(60) Divisional application: 11166139.3
(73) Proprietor: Evolva SA, 4153 Reinach (CH)
(72) Inventor: KRISTIANSEN, Karsten, 5672 Brody (DK); MAINKAR, Prathama S., Hyderabad 500 027, AP (IN); MEYER, Jean-Philippe, 67000 Strasbourg (FR); SANTANA SORENSEN, Alexandra c/o Evolva SA, 4153 Reinach (CH)
(74) Representative: Crease, Devanand John
(86) International application number: PCT/IB2007/002542
(87) International publication number: WO 2007/138485

(56) References cited:
- EP-A- 0 094 239
- WO-A-00/30683
- WO-A2-02/38157
- US-A- 5 248 780
- BROWN G R, CLARKE D S, FAULL A W, FOUBISTER A J, SMITHERS M J: "Design of dual-acting thromboxane antagonist-synthase inhibitors by a mutual prodrug approach" BIOORGANIC & MEDICINAL CHEMISTRY LETTERS, vol. 6, no. 3, 1996, pages 273-278, XP002461929
- BREWSTER A G, BROWN G R, FOUBISTER A J, JESSUP R, SMITHERS M J: "The synthesis of a novel thromboxane receptor antagonist 4(Z)-6-(2-o-chlorophenyl-4-o-hydroxyphenyl -1,3-dioxan-cis-5-yl) hexenoic acid ICI 192,605" PROSTAGLANDINS, vol. 36, no. 2, August 1988 (1988-08), pages 173-178, XP002461930
- BREWSTER A G, CAULKETT P W R, JESSUP R: "(5Z)-7-(2,2-dimethyl-4-phenyl-1,3-dioxan- cis-5-yl)heptenoic acid: A specific thromboxane A2 receptor antagonist" JOURNAL OF MEDICINAL CHEMISTRY, vol. 30, 1987, pages 67-70, XP002461931
- BROWN G R, FOUBISTER A J: "ICI-180080 A NOVEL SELECTIVE THROMBOXANE RECEPTOR ANTAGONIST SYNTHESIS AND RELATIVE ACTIVITY" JOURNAL OF PHARMACY AND PHARMACOLOGY, vol. 38, no. 9, 1986, pages 706-708, XP009093488
- HALL S E: "THROMBOXANE A2 RECEPTOR ANTAGONISTS" MEDICINAL RESEARCH REVIEWS, NEW YORK, NY, US, vol. 11, no. 5, 1991, pages 503-579, XP002923626 ISSN: 0198-6325

## Description

### Field of invention

The present invention is directed to 2,4-diphenyl-1,3-dioxanes for use in the treatment of a disease or condition dependent on PPAR modulation, such as diabetes, cancer, inflammation, neurodegenerative disorders and infections.

### Background of invention

Peroxisome proliferator-activated receptors (PPAR) are nuclear hormone receptors. PPAR receptors activate transcription by binding to elements of DNA sequences, known as per-oxisome proliferator response elements (PPRE), in the form of a heterodimer with retinoid X receptors (known as RXRs). Three sub-types of human PPAR have been identified and described: PPAR-alpha, PPAR-gamma and PPAR- delta (or NUCI). PPAR-alpha is mainly expressed in the liver, while PPAR-delta is ubiquitous. PPAR-gamma is involved in regulating the differentiation of adipocytes, where it is highly expressed. It also has a key role in systemic lipid homeostasis. A number of compounds that modulate the activity of PPARs have been identified including thiazolidinediones, which have been employed in the treatment of diabetes.

The DNA sequences of the PPAR-gamma subtypes are described in Elbrecht et al., BBRC 224; 431-437 (1996). Peroxisome proliferators including fibrates and fatty acids activate the transciptional activity of PPARs.

Numerous examples are provided in the literature illustrating that PPARs are closely involved in a wide array of diseases or pathological conditions which are associated with cells expressing these nuclear receptors. More specifically, PPARs are useful as drug target in methods for reducing blood glucose, cholesterol and triglyceride levels and are accordingly explored for the treatment and/or prophylaxis of insulin resistance, dyslipidemia, and other disorders related to Syndrome X (also designated "the metabolic syndrome) (WO 97/25042, WO 97/10813, WO 97/28149; see also Kaplan et al., 2001, J Cardiovasc Risk, 8, 211-7) including obesity and atherosclerosis (Duez et al., 2001, J. Cardiovasc. Risk, 8,185-186), coronary artery disease and certain other cardiovascular disorders. Further, PPARs have been shown to be potential targets for the treatment of certain inflammatory diseases such as cutaneous disorders (see Smith et al., 2001, J. Cutan. Med. Surg., 5,231-43), gastrointestinal diseases (WO 98/43081) or renal diseases including glomerulonephritis, glomerulosclerosis, nephrotic syndrome and hypertensive nephrosclerosis. Similarly PPARs are useful for improving cognitive functions in neurologic diseases (Landreth and Heneka, 2001, Neurobiol Aging, 22,937-44) or in dementia, for treating psoriasis, polycystic ovarian syndrome (PCOS) or for preventing and treating bone loss, e. g. osteoporosis (see for example US 5,981,586 or US 6,291,496).

Thus, PPARs are exciting targets for the development of therapeutic compounds. Although, the responses observed in the context of the various methods for treating and/or preventing diseases or pathological conditions are encouraging (for example, the thiazolidinedione (TZD) class of medications, e. g. troglitazone, rosiglitazone or pioglitazone, unambiguously plays a critical role in improving insulin sensitivity in patients with type 2 diabetes; see Cheng lai and Levine, 2000, Heart Dis., 2,326-333), they are not fully satisfactory treatments because of the occurrence of numerous serious undesirable side effects (for example, weigh gain, hypertension, cardiac hypertrophy, haemodilution, liver toxicity and oedema; see Haskins et al., 2001, Arch Toxicol., 75,425-438; Yamamoto et al.,2001, Life Sci., 70,471-482; Scheen, 2001, Diabetes Metab., 27,305-313; Gale, 2001, Lancet, 357,1870-1875;Forman et al., 2000, Ann. Intern. Med., 132,118-121 and Al Salman et al., 2000, Ann. Intern. Med., 132,121-124). Consequently, it is desirable to identify novel improved products and/or novel methods which enable the treatmentand/or the prevention of diseases or pathological conditions associated with cell types that express PPAR nuclear receptors. More specifically, most of the side effects observed with TZD derivatives are attributable to the full-agonist properties of said compounds and thus it is desirable to identify new compounds that are not necessarily full-agonists.

Certain 4-phenyl-1,3-dioxan-5-ylalkenoic acid derivatives have been described as thromboxane receptor antagonists or inhibitors of thromboxane A2 synthesis. The thromboxane receptor is a potent aggregator of blood platelets and has been implicated in vasoconstriction, as well as in bronchial and tracheal smooth muscle constriction (see for example European patent application, publication No. 94239; European Patent application Publication No.0 266 980 and USPN 4 895 962).

### Summary of the invention

One aspect of the invention relates to 1,3-dioxanes for use in a method for modulating the activity of a PPAR in an individual comprising administering a therapeutically effective amount of

a 1,3-dioxane derivative or a pharmaceutically acceptable salt thereof, wherein the derivative is represented by the formula: and exhibits a cis-stereochemistry around the dioxane ring in positions 2, 4 and 5: 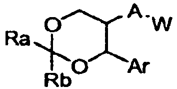 wherein
A is a branched or linear carbon chain of 3 to 7 carbons with up to 2 double bonds;
W is COOH, OH, NH₂, SO₃H, OSO₃H, or an aromatic group selected from the group consisting of phenyl, 1- or 2-naphthyl, pyridine, furan, 2-methylpyridine and a dioxolane, each optionally substituted with COOH, OH or NH₂
Ar is a phenyl, or a 5 or 6 membered heterocyclic aromatic group selected from substituted or unsubstituted 2-pyridine, 3-pyridine, thiophene, furan, 1-naphthyl, 2-naphthyl, biphenyl and (4-methoxyphenoxy)-phenyl
and,
Ra and Rb are independently hydrogen, 2-6C alkenyl, 1-8C alkyl optionally with up to three halogeno substiuents, pentafluorophenyl, aryl or aryl(1-4C)alkyl, said aryl or aryl(1-4C) alkyl substituents being optionally substituted with halogeno, (1-6C)alkyl, branched or linear (1-6C)alkoxy, (1-4C)alkylenedioxy, trifluoromethyl, cyano, nitro, hydroxyl, (2-6C)alkanoyloxy, (1-6C)alkylthio, (1-6C)alkanesulphonyl, (1-6C)alkanoylamino and oxapolymethylene of 2 to 4 carbon atoms, or Ra and Rb together form polymethylene of 2 to 7 carbon atoms, optionally having one or two (1-4C)alkyl substituents.

Also provided are 2,4-diphenyl-1,3-dioxane derivatives for use in methods for treating PPAR responsive diseases or conditions by administering a 2,4-diphenyl-1,3-dioxane derivative or a pharmaceutically acceptable salt thereof, wherein the derivative is represented by formula II: 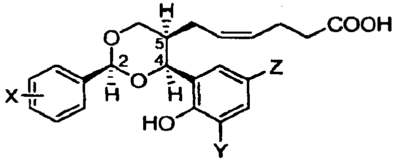 wherein X is selected from fluoro, chloro, bromo, trifluoromethyl, optionally substiuted phenyl, cyano, methoxy and nitro, or the phenyl-X group can be an optionally substituted chromen derivative; and Y and Z are individually hydrogen or halogeno.

Also provided by the invention 1,3-dioxane derivatives, their pharmaceutically acceptable salts and pharmaceutical compositions comprising the derivatives represented by the formula: and exhibiting a cis-stereochemistry in positions 2, 4 and 5 of the dioxane ring: 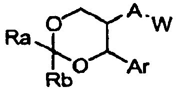 wherein:
A is a branched or linear carbon chain of 3 to 7 carbons with up to 2 double bonds;
W is COOH, OH, NH₂, SO₃H, OSO₃H, or an aromatic group selected from the group consisting of phenyl, 1- or 2-naphthyl, pyridine, furan, 2-methylpyridine and a dioxolane, each optionally substituted with COOH, OH or NH₂
Ar is a phenyl, or a 5 or 6 membered heterocyclic aromatic group selected from substituted or unsubstituted 2-pyridine, 3-pyridine, thiophene, furan, 1-naphthyl, 2-naphthyl, biphenyl and (4-methoxyphenoxy)-phenyl
and,
Ra is H and Rb is an aryl group or a heterocycle optionally substituted with three different substituents selected from the group consisting of halogen, OH, O-alkyl, O-aryl, amino or N-monoalkyl or N-dialkyl or N-monoaryl or N-diaryl, nitro, thioalkyl or oxo. Particular compounds of interest are 4(Z)-6-(2-[4-methoxyphenoxy-o-phenyl]-4-o-hydroxyphenyl-1,3-dioxan-cis-5-yl)hexenoic acid and 4(Z)-6-(2-3-[6-chloro-4H-chromen-4-one]-4-o-hydroxyphenyl-1,3-dioxan-cis-5-yl)hexenoic acid, or compounds where Rb is biphenyl.

The invention also relates to uses of compounds as defined above, for the preparation of a medicament for the treatment of diseases or conditions which are responsive to modulation of PPAR-gamma activity, in particular any of the PPAR-gamma responsive diseases or conditions described herein below.

The compounds are useful in the preparation of a medicament for treatment or prevention of a number of clinical conditions including the metabolic syndrome, obesity, insulin resistance, pre-diabetes, diabetes, dyslipidemia, and wound healing.

### Description of the drawings

Figure 1 illustrates a model of PPAR activation by a full agonist and a partial agonist, respectively.
Figure 2 illustrates selective activation of PPAR-gamma by rosiglitazone and 4(Z)-6-(2-o-chlorophenyl-4-o-hydroxyphenyl-1,3-dioxan-cis-5-yl)hexenoic acid, compared with PPAR-delta, PPAR alpha and RxR.
Figure 3 illustrates activation of full length PPAR-gamma by rosiglitazone and 4(Z)-6-(2-o-chlorophenyl-4-o-hydroxyphenyl-1,3-dioxan-cis-5-yl)hexenoic acid.
Figure 4 illustrates that 4(Z)-6-(2-o-chlorophenyl-4-o-hydroxyphenyl-1,3-dioxan-cis-5-yl)hexenoic acid has no effect on full length PPAR delta transactivation.
Figure 5 illustrates results of partial PPARgamma competition assays.
Figure 6 illustrates results of PPARgamma ligand displacement assays.
Figure 7 illustrates results of glucose uptake assays.

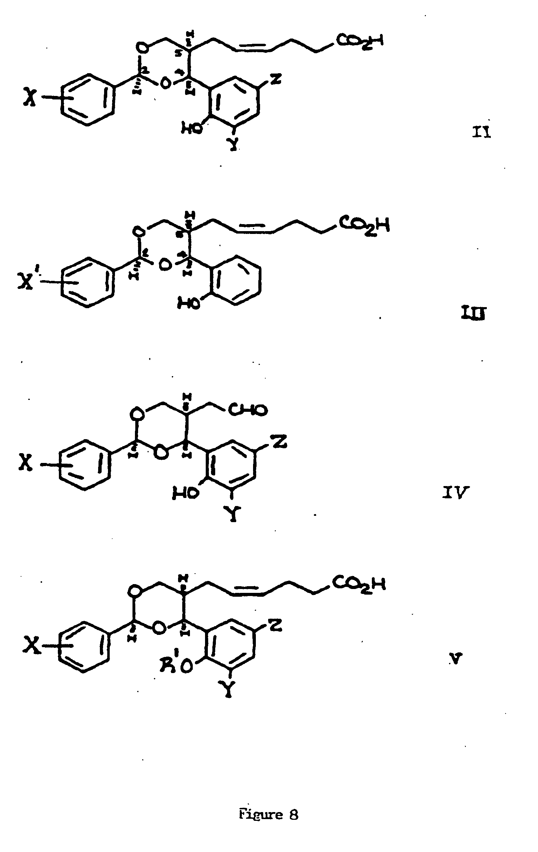

Figure 8 illustrates chemical formulae II -V.

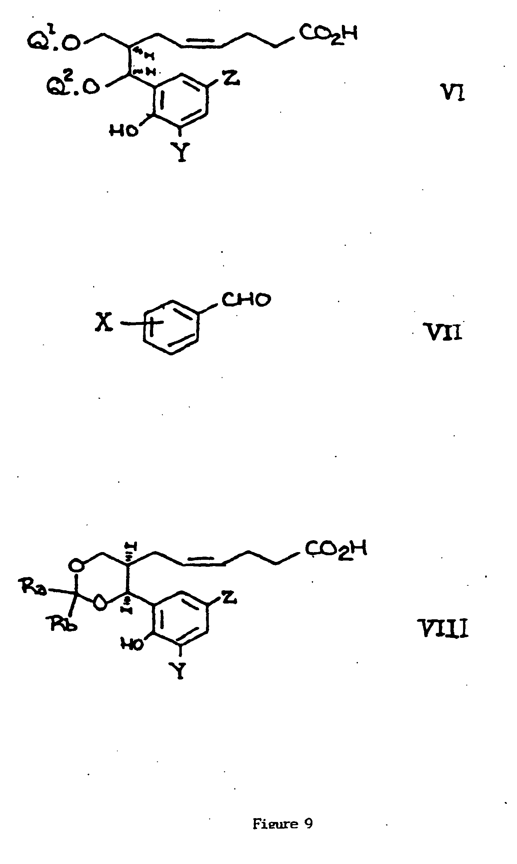

Figure 9 illustrates chemical formulae VI-VIII.

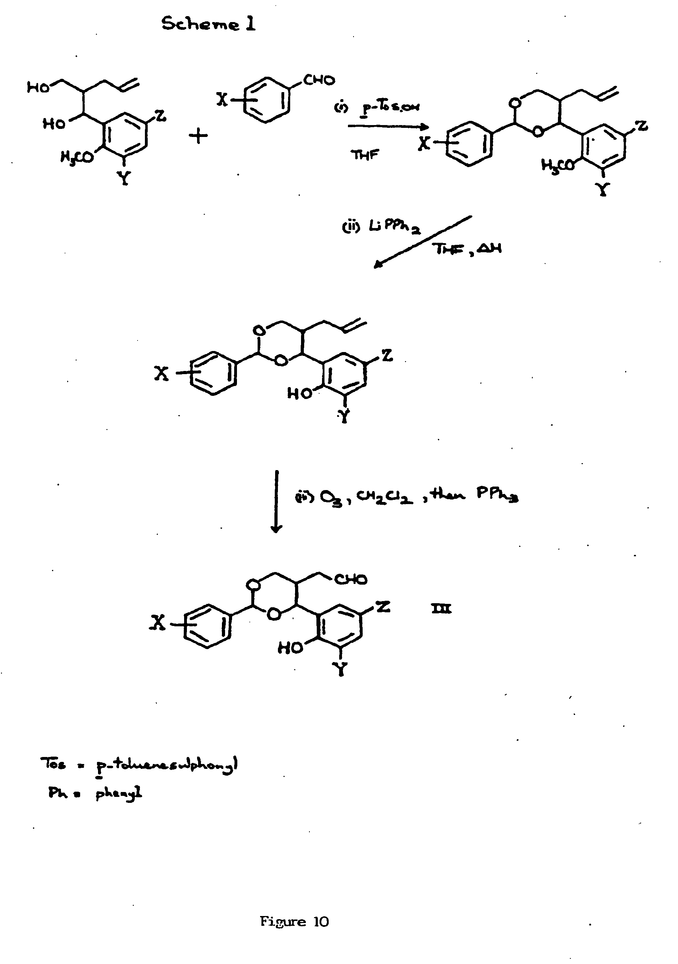

Figure 10 illustrates chemical Reaction Scheme I.

### Detailed description of the invention

### Definitions

In general the terms used herein will take their standard definitions that one of ordinary skill in the pharmaceutical, biological and chemical arts would employ in understanding the invention. The following terms have the meanings given, and other terms may be provided in the specifications.

Alkyl: The term "alkyl" refers to a monovalent, saturated aliphatic hydrocarbon radical having the indicated number of carbon atoms, generally one to twenty two. For example, a "1-8 C alkyl" or an "alkyl of 1-8 carbons" or "Alk 1-8" would refer to any alkyl group containing one to eight carbons in the structure. Alkyl may be a straight chain (i.e. linear) or a branched chain. Lower alkyl refers to an alkyl of 1-6 carbons. Representative examples of lower alkyl radicals include methyl, ethyl, n-propyl, n-butyl, n-pentyl, n-hexyl, isopropyl, isobutyl, isopentyl, amyl, sec-butyl, tert-butyl, sec-amyl, tert-pentyl, 2-ethylbutyl, 2,3-dimethylbutyl, and the like. Higher alkyl refers to alkyls of seven carbons and above. These include n-heptyl, n-octyl, n-nonyl, n-decyl, n-dodecyl, n-tetradecyl, n-hexadecyl, n-octadecyl, n-eicosyl, and the like, along with branched variations thereof. A linear carbon chain of 3 to 7 carbons would refer to the chain length, not including any carbons residing on a branch. The radical may be optionally substituted.

Alkenyl: The term "alkenyl" refers to a monovalent, aliphatic hydrocarbon radical having at least one carbon-carbon double bond and having the indicated number of carbon atoms. For example, a "C 2-6 alkenyl" or an "alkenyl of 1-6 carbons," or "alkenyl 1-6" would refer to an alkenyl group containing one to six carbon atoms in the structure. Alkenyl may be a straight chain (i.e., linear) or a branched chain. Lower alkenyl refers to an alkenyl of 1-6 carbons. Representative examples of lower alkenyl radicals include ethenyl, 1-propenyl, 1-butenyl, 1-pentenyl, 1-hexenyl, isopropenyl, isobutenyl, and the like. Higher alkenyl refers to alkenyls of seven carbons and above. These include 1-heptenyl, 1-octenyl, 1-nonenyl, 1-decenyl, 1-dodecenyl, 1-tetradecenyl, 1-hexadecenyl, 1-octadecenyl, 1-eicosenyl, and the like, along with branched variations thereof. The radical may be optionally substituted.

Alkoxy: The term "alkoxy" refers to a monovalent radical of the formula RO-, where R is an alkyl as defined herein. Lower alkoxy refers to an alkoxy of 1-6 carbon atoms or (1-6)alkoxy. Representative lower alkoxy radicals include methoxy, ethoxy, n-propoxy, n-butoxy, n-pentyloxy, n-hexyloxy, isopropoxy, isobutoxy, isopentyloxy, amyloxy, sec-butoxy, tert-butoxy, tert-pentyloxy, and the like. The radical may be optionally substituted
Aryl: The term "aryl" as used herein denotes a monovalent aromatic carbocyclic radical containing 5 to 15 carbon atoms consisting of one individual ring, or one or more fused rings in which at least one ring is aromatic in nature, which can optionally be substituted with one or more, preferably one or three substituents independently selected from hydroxy, thio, cyano, alkyl, alkoxy, lower haloalkoxy, alkylthio, oxo, halogen, haloalkyl, hydroxyalkyl, nitro, alkoxycarbonyl, amino, alkylamino, dialkylamino, aminoalkyl, alkylaminoalkyl, and dialkylaminoalkyl, thioalkyl, alkylsulfonyl, arylsulfinyl, alkylaminosulfonyl, arylaminosulfonyl, alkylsulfonylamino, arylsulfonylamino, carbamoyl; alkylcarbamoyl and dialkylcarbamoyl, arylcarbamoyl, alkylcarbonylamino, arylcarbonylamino, unless otherwise indicated. Alternatively two adjacent atoms of the aryl ring may be substituted with a methylenedioxy or ethylenedioxy group. Thus a bicyclic aryl substituents may be fused to a heterocyclyl or heteroaryl ring; however, the point of attachment of bicyclic aryl substituent is on the carbocyclic aromatic ring. Examples of aryl radicals include, phenyl, naphthyl, benzyl, biphenyl, furanyl, pyridinyl, indanyl, anthraquinolyl, tetrahydronaphthyl, 3.4-methylenedioxyphenyl, 1,2,3,4-tetrahydroquinolin-7-yl, 1,2,3,4-tetrahydroisoquinoline-7-yl, a 1,3 dioxolane radical, a benzoic acid radical, , a furan-2-carboxylic acid radical, a 2-(isoxazol-5-yl)acetic acid radical, a 3-hydroxy-2-methylpyridine-4-carboxylic acid radical and the like.
Halo: A "halo" substitutent is a monovalent halogen radical chosen from chloro, bromo, iodo, and fluoro. A "halogenated" compound is one substituted with one or more halo substituent.
Phenyl: A "phenyl" is a radical formed by removal of a hydrogen from a benzene ring. The phenyl may be optionally substituted.
Phenoxy: A "phenoxy" group is a radical of the formula RO-, wherein the R is a phenyl radical.
Benzyl: A "benzyl" group is a radical of the formula R-CH₂-, wherein the R is a phenyl radical.
Benzyloxy: A "benzyloxy" group is a radical of the formula RO-, wherein R is a benzyl radical.
Heterocycle: A "heterocycle" or "heterocyclic entity"is a monovalent radical of a 5- or 6-member closed ring containing carbon and at least one other element, generally nitrogen, oxygen, or sulfur and may be fully saturated, partially saturated, or unsaturated (i.e., aromatic in nature). Generally the heterocycle will contain no more than two hetero atoms. Representative examples of unsaturated 5-membered heterocycles with only one hetero atom include 2- or 3-pyrrolyl, 2- or 3-furanyl, and 2- or 3-thiopenyl. Corresponding partially saturated or fully saturated radicals include 3-pyrrolin-2-yl, 2- or 3-pyrrolindinyl, 2- or 3-tetrahydrofuranyl, and 2- or 3-tetrahydrothiophenyl. Representative unsaturated 5-membered heterocyclic radicals having two hetero atoms include imidazolyl, oxazolyl, thiazolyl, pyrazolyl, and the like. The corresponding fully saturated and partially saturated radicals are also included. Representative examples of unsaturated 6-membered heterocycles with only one hetero atom include 2-, 3-, or 4-pyridinyl, 2H-pyranyl, and 4H-pryanyl. Corresponding partially saturated or fully saturated radicals include 2-, 3-, or 4-piperidinyl, 2-, 3-, or 4-tetrahydropyranyl and the like. Representative unsaturated 6-membered heterocyclic radicals having two hetero atoms include 3- or 4-pyridazinyl, 2-, 4-, or 5-pyrimidinyl, 2-pyrazinyl, morpholino, and the like. The corresponding fully saturated and partially saturated radicals are also included, e.g. 2-piperazine. The heterocyclic radical is bonded through an available carbon atom or hetero atom in the heterocyclic ring directly to the entity or through a linker such as an alkylene such as methylene or ethylene. The heterocycle may be optionally substituted in the same way as aryl groups.
Optionally substituted: If a radical is referred to as "optionally substituted," it means that the radical is unsubstituted or at least one-H of the radical is removed and another substituent inserted in its place. The radical may be optionally substituted with substituents at positions that do not significantly interfere with the preparation of compounds falling within the scope of this invention and that do not significantly adversely affect the biological activity of the compounds. The radical is optionally substituted with one, two, three, four or five substituents independently selected from the group consisting of halo, lower alkoxy, hydroxyl, cyano, nitro, amino, halo lower alkyl, halo lower alkoxy, hydroxycarbonyl, lower alkoxycarbonyl, lower alkylcarbonyloxy, and lower alkylcarbonylamino, or as referred to hereinabove.

The term "hydroxycarbonyl" is a monovolent radical having the formula -C(O)OH.

The term "lower alkoxycarbonyl" is a monovalent radical with the formula -C(O)OAlk, where Alk is lower alkyl.

The term "lower alkylcarboxyloxy" is a monovalent radical with the formula -OC(O)Alk, where Alk is lower alkyl.

As used herein, "a sugar " means a monosaccharide, a disaccharide or a polysaccharide. Suitable monosaccharides include pentose, hexose, or a heptose residues. Non-limiting examples of pentoses include arabinose, ribose, ribulose, xylose, lyxose, and xylulose. Non-limiting examples of hexoses include glucose, galactose, fructose, fucose, mannose, allose, altrose, talose, idose, psicose, sorbose, and tagatose. Non-limiting examples of heptoses include mannoheptulose and sedoheptulose. A sugar moiety may be linked to the compound at any position of the sugar ring which can form an amide or ester bond. Preferred saccharides are beta-glycosyl saccharides.

PPAR modulation is defined by reference to the natural situation, i. e. the basal level of PPAR dependent transcription of target genes in the absence of ligands, wherein modulation of PPAR activity is reflected by decrease or increase in said basal level of transcription in the presence of a compound capable of modulating PPAR activity. Generally, an increase of said transcription is associated with an enhancement of PPAR activity and relates to compounds named activators or agonists. Conversely, a decrease of said transcription is associated with an inhibition of PPAR activity and relates to compounds named inhibitors or antagonists. Partial agonists are compounds that result in PPAR dependent transcription of a subset of target genes while having no effect on other PPAR target genes. Partial agonists may be viewed from biochemical or physiological viewpoint. The biochemical view of a partial agonist is a compound that can compete out a full agonist and has a lower level of transactivation relative to a full agonist. The physiological view of a partial agonist relates to the activation of different subsets of genes (even full activation of some target genes and no activation of other PPAR target genes). This results in only some of the physiological effects of a full agonist, which is highly desirable.

A "therapeutically effective amount" of compound means the amount that, when administered to a subject in need thereof, will produce the desired result over time for the condition being treated. In this application, the desired effect is PPAR modulation and the biological activity associated therewith.

The compounds useful in this invention are depicted by various formulae in this application. By viewing the formulae, it will be apparent that the compounds often will have a chiral center, i.e., a carbon to which four different groups are attached, and these can exist as enantiomers. In addition, because of the presence in some cases of double bonds, a compound will have hindered rotation. The compound thus exhibits geometric isomerism, i.e. two forms can differ from each other in the way the atoms are oriented in space. With regard to the double bond, stereoisomers exist that are not mirror images of each other, and are referred to as diastereomers. In naming the compounds in this application, the Chemical Abstracts Service system is used in which the two groups attached to each end of the double bond are provided a priority number as is done in naming enantiomers in the R, S system. When two groups of the higher priority number are on the same side, the molecule is the Z isomer. (German-*zusammen*, together). The molecule is E when on opposite sides (German-*entgegen,* opposite).

The compounds of the invention exhibit a cis-stereochemistry in positions 2, 4 and 5 of the dioxane ring.

### Compounds useful in the invention

The invention is based in part on the discovery that certain compounds are capable of modulating the activity of at least one PPAR subtype, for example PPAR gamma or PPAR beta/delta. This discovery leads to the use of these compounds to treat conditions or diseases in mammals, such as humans, mediated by the function of such PPARs.

The compounds that are useful in this invention are1,3-dioxane derivatives or a pharmaceutically acceptable salt thereof, wherein the derivative is represented by formula I: and exhibits a cis-stereochemistry at positions 2, 4 and 5 of the dioxane ring: 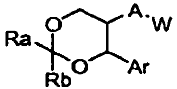 wherein
A is a branched or linear carbon chain of 3 to 7 carbons, optionally containing 1 or 2 double bonds (each can be cis or trans)
W is COOH, OH, NH₂, SO₃H, OSO₃H, an aromatic group such as, but not limited to, phenyl, 1- or 2-naphthyl, pyridine, furan, 2-methylpyridine, optionally substituted with COOH, OH or NH₂, for example; or a 1,3 dioxolane group linked through the 2 position
Ar is a phenyl, or a 5 or 6 membered heterocyclic aromatic group, such as, but not limited to, 2-pyridine, 3-pyridine, thiophene, furan, 1-naphthyl, 2-naphthyl, biphenyl and (4-methoxyphenoxy)-phenyl, the phenyl and naphthyl moieties optionally substituted with OH or OMe, in the ortho, meta and/or para position, but preferably if substituted, mono substituted in the ortho position with OH or OMe
and,
Ra and Rb are independently hydrogen, 2-6C alkenyl, 1-8C alkyl optionally with up to three halogeno substiuents, pentafluorophenyl, aryl or aryl(1-4C)alkyl, the latter two of which may optionally have up to five substituents selected from halogeno, (1-6C)alkyl, branched or linear (1-6C)alkoxy, (1-4C)alkylenedioxy, trifluoromethyl, cyano, nitro, hydroxyl, (2-6C)alkanoyloxy, (1-6C)alkylthio, (1-6C)alkanesulphonyl, (1-6C)alkanoylamino and oxapolymethylene of 2 to 4 carbon atoms, or Ra and Rb together form polymethylene of 2 to 7 carbon atoms, optionally having one or two (1-4C)alkyl substituents.

In some embodiments, A is a linear carbon chain of 3 to 7 carbons, and W is COOH. Such a carbon chain may include a double bond between C2-C3 or C3-C4, for example.

In one embodiment Ra is H and Rb is an aromatic moiety such as phenyl, benzyl, 2- or 3- or 4-pyridine, furan, biphenyl, 1- or 2-naphthyl, bearing up to five different substituents selected from the group consisting of halogen, OH, O-alkyl, amino, N- monoalkyl, N-dialkyl (branched or linear), nitro and thioalkyl (branched or linear).

In some embodiments, the derivative is a 2,4-diphenyl-1,3-dioxane derivative or a pharmaceutically acceptable salt thereof, wherein the derivative is represented by formula II: 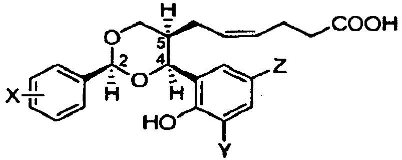 wherein X is selected from fluoro, chloro, bromo, trifluoromethyl, optionally substiuted phenyl, cyano, methoxy and nitro, or the phenyl-X group can be an optionally substituted chromen derivative; and Y and Z are individually hydrogen or halogeno.

Typically, the groups at positions 2, 4 and 5 of the dioxane ring in the derivative represented by formula I will have cis-relative stereochemistry.

Other specific substitutions of the phenyl moeity bearing X which are of particular interest include, for example 2-fluoro-, 2-chloro-, 2-bromo-, 2-cyano-, 2-trifluoromethyl-, 3-fluoro-, 3-chloro-, 3-cyano-, 3-nitro-, 3-methoxy-, 4-chloro-, 4-cyano-, 4-nitro- and 4-methoxy-phenyl.

A preferred substitution for Y is hydrogen or fluoro and for Z is hydrogen.

A preferred group of compounds useful in the invention comprises those compounds of formula III (set out hereinafter) wherein X¹ is selected from 2-chloro, 3-chloro, 2-cyano, 4-cyano, 3-nitro and 4-nitro; and the groups at positions 2, 4 and 5 of the dioxane ring have cis- relative stereochemistry; together with the pharmaceutically acceptable salts thereof.

The invention also provides compounds of formula I, where A, W and Ar are defined as above and wherein Ra is H and Rb is an aryl group or a heterocycle, such as where one carbon atom of the aryl moiety is replaced by one oxygen or nitrogen atom. Such aryl and heterocycle groups can be optionally substituted with three different substituents selected from the group consisting of halogen, OH, O-alkyl (branched or linear), O-aryl, amino or N-monoalkyl or N-dialkyl (branched or linear) or N-monoaryl or N-diaryl, nitro, thioalkyl (branched or linear) or oxo (e.g SN13 in Table II). Compounds of formula I where Ra is H and Rb is a heterocycle or a phenyl group substituted with 0-aryl are of particular interest as PPAR gamma modulators, especially when Ar is phenyl substituted in the o-position by OH or OMe, W is COOH and A is a five carbon linear chain with one double bond. Illustrative examples of such compounds include compounds of formula I, where Ra is H and Rb is (4-methoxyphenoxy)-phenyl linked to the dioxane ring through the 2 or ortho position of the phenyl, such as 4(Z)-6-(2-[4-methoxyphenoxy-o-phenyl]-4-o-hydroxyphenyl-1,3-dioxancis-5-yl)hexenoic acid; or Rb is 6-chloro-4H-chromen-4-one (for example, linked to the dioxane ring through the 3 position of the chromene moiety), such as 4(Z)-6-(2-3-[6-chloro4H-chromen-4-one ]-4-o-hydroxyphenyl-1,3-dioxan-cis-5-yl)hexenoic acid; or Rb is biphenyl, linked to the dioxane ring through the 2 or ortho position of biphenyl. Thus the invention provides the compounds referred to in this embodiment and their pharmaceutically relevant salts, alone and in combination with a pharmaceutical carrier and optionally with a further therapeutically active ingredient.

It will be appreciated that the compounds of formula I and formula II possess asymmetric carbon atoms and may exist and be isolated in racemic and optically active forms. The invention includes compounds which exhibit a cis-stereochemistry at positions 2, 4 and 5 of the dioxane ring and which are capable of modulating PPAR activity, and their uses, it being well known in the art how to prepare individual optical isomers (for example by synthesis from optically active starting materials or chromatographic resolution of a racemic form) and how to determine PPAR modulating properties using one or more of the assays referred to hereafter. Unless otherwise clear from the context, the chemical formulae referred to herein may be shown in a particular configuration, but this does not necessarily correspond to the absolute configuration.

Particular pharmaceutically acceptable salts of acids of formula I or II are, for example, alkali metal and alkaline earth metal salts such as lithium, sodium potassium, magnesium and calcium salts, aluminium and ammonium salts, and salts with organic amines and quaternary bases forming physiologically acceptable cations such as salts with methylamine, dimethylamine, trimethylamine, ethylenediamine, piperidine, morpholine, pyrrolidine, piperazine, ethanolamine, triethanolamine, N-methylglucamine, tetramethylammonium hydroxide and benzyltrimethylammonium hydroxide.

The compounds of formula I may be manufactured by conventional procedures of organic chemistry well known in the art for the manufacture of structurally analogous compounds. Such procedures are provided for example in EP 0 094 239 pages 4 - 10, and are illustrated below in the Examples section and by the following processes in which X, Y and Z have any of the meanings defined hereinabove:
(A) An aldehyde of the formula IV is reacted with a Wittiig reagent of the formula R¹₃ P=CH(CH₂)₂CO₂⁻M⁺ wherein R¹ is (1-6C)alkyl or aryl (especially phenyl) and M⁺ is a cation, for example an alkali metal cation such as the lithium, sodium or potassium cation. In some embodiments the aldehyde will be reacted with a Wittig reagent of the formula be modified to P=CH(CH₂)ₙCOO⁻M⁺ where n=0 to 4.
   The process in general produces the required compounds of formula II in which the substituents adjacent to the double bond have predominantly *cis*-relative stereochemistry i.e. the "Z" isomer. However the process also produces analogous compounds having *trans-*relative stereochemistry which may be removed by a conventional procedure such as chromatography or crystallisation, if desired.
   The synthetic process is conveniently performed in a suitable solvent or diluent, for example an aromatic solvent such as benzene, toluene or chlorobenzene, an ether such as 1,2-dimethoxyethane, t-butyl methyl ether, dibutyl ether or tetrahydrofuran, in dimethyl sulphoxide or tetramethylene sulphone, or in a mixture of one or more such solvents or diluents. The process is generally performed at a temperature in the range, for example, of -80° C to 40° C, but is conveniently performed at or near room temperature, for example in the range 0 to 35° C.
(B) A phenol derivative of the formula V wherein R¹ is a protecting group, for example (1-6C) alkyl (such as methyl or ethyl), acyl (such as acetyl, benzoyl, methanesulphonyl or p-toluenesulphonyl), allyl, tetrahydropyran-2-yl, trimethylsilyl, and is deprotected.
   The deprotection conditions used depend on the nature of the protecting group R¹. Thus, for example, when it is methyl or ethyl the deprotection may be carried out by heating with sodium thioethoxide (hydride and ethanethiol) in a suitable solvent (such as N,N-dimethylformamide or N,N-dimethyl-3,4,5,6-tetrahydro-2(1H)-pyrimidinone) at a temperature in the range, for example, of 50 to 160 C. Alternatively, an ethyl or methyl protecting group may be removed by reaction with lithium diphenylphosphide in a suitable solvent (such as tetrahydrofuran or methyl t-butyl ether) at a temperature in the range, for example, of 0 to 60° C. When the protecting group is acyl it may be removed, for example, by hydrolysis in the presence of a base (such as sodium or potassium hydroxide) in a suitable aqueous solvent [such as an aqueous (1-4C) alkanol] at a temperature in the range, for example, of 0 to 60° C. When the protecting group is allyl or tetrahydropyran-2-yl, it may be removed, for example, by treatment with strong acid such as trifluoroacetic acid and when it is trimethylsilyl, it may be removed, for example, by reaction with aqueous tetrabutyl ammonium fluoride or sodium fluoride using a conventional procedure.
(C) An erythro-diol derivative of the formula V wherein one of Q¹ and Q² is hydrogen and the other is hydrogen or a group of the formula -CRaRb.OH (wherein Ra and Rb are the same or different (1-4C) alkyl) is reacted with a benzaldehyde derivative of the formula VII or an acetal, hemiacetal or hydrate thereof.

The benzaldehyde VII [or its hydrate, or its acetal or hemiacetal with a (1-4C)alkanol (such as methanol or ethanol)] may conveniently be present in an excess.

The reaction is generally performed in the presence of an acid catalyst such as hydrogen chloride, hydrogen bromide, sulphuric acid, phosphoric acid, methanesulphonic acid or p-toluenesulphonic acid, conveniently in the presence of a suitable solvent or diluent, such as toluene, xylene or an ether, for example tetrahydrofuran, dibutyl ether, methyl t-butyl ether or 1,2-dimethoxyethane, and at temperature in the range, for example, of 0 to 80° C.

Those starting materials of formula VI wherein Q¹ and Q² are both hydrogen may be obtained, for example, by mild, acid catalysed, hydrolysis or alcoholysis of the dioxane ring of a compound of formula VIII wherein Ra and Rb are both alkyl such as methyl or ethyl, obtained by an analogous procedure to process (A) herein. The hydrolysis or alcoholysis will normally be carried out at a temperature in range of 10 to 80° C. using an aqueous mineral acid such as hydrochloric acid in an alkanol (such as ethanol or 2-propanol) or an ether (such as tetrahydrofuran) as solvent.

The starting materials of formula VI wherein one of Q¹ and Q² is hydrogen and the other is a group of the formula --CRaRb.OH are intermediates in the above-mentioned formation of the starting materials of formula VI wherein Q¹ and Q² are both hydrogen. However, said intermediates are not normally isolated or characterised. Therefore, a useful modification of process (C) comprises reacting a compound of formula VIII wherein one of Ra and Rb is hydrogen, methyl or ethyl and the other is methyl or ethyl with an excess of a compound of the formula VII (or a hydrate, acetal or hemiacetal thereof) in the presence of an acid catalyst (such as one of those given above), conveniently at a temperature in the range, for example, of 10 to 80° C, and optionally in the presence of a suitable solvent or diluent (such as one of those given above).

The starting materials for use in the above processes may be made by general procedures of organic chemistry, known for the preparation of structurally related compounds. Thus, the aldehydes of formula IV may be obtained, for example, by the method shown in Scheme I. The protected phenol derivatives of formula V may be made, for example, by using an analogous procedure to process (A) above using an aldehyde analogous to that of formula IV, but wherein the phenol group has been protected with the group R¹, such an aldehyde being made, for example, by carrying out the procedures of Scheme I omitting the deprotection step (ii). Those of the starting materials of formula VIII which are novel may be obtained using analogous procedures to those described in European patent application, publication No. 94239.

The necessary Wittig reagents may be obtained by conventional procedures, for example by treating the corresponding phosphonium halides with a strong base such as sodium hydride, lithium diisopropylamide, potassium t-butoxide, LiHMDS or butyllithium. They are generally formed in situ just prior to carrying out the condensation process (A) above.
It will be understood that the compounds of formula I and II may also be obtained by other conventional procedures well known in the art, for example by base catalysed hydrolysis of the corresponding esters, amides or nitriles.

When a salt of a compound of formula I or II is required, it is obtained by reaction with the appropriate base affording a physiologically acceptable cation, or by any other conventional procedure.

Further, when an optically active form of a compound of formula I or II is required, one of the aforesaid processes may be carried out using an optically active starting material. Alternatively, the racemic form of a compound of formula II may be reacted with an optically active form of a suitable organic base, for example ephedrine, N,N,N-trimethyl(1-phenylethyl)ammonium hydroxide or 1-phenylethylamine, followed by conventional separation of the diastereoisomeric mixture of salts thus obtained, for example by fractional crystallisation from a suitable solvent, for example a (1-4C) alkanol, whereafter the optically active form of said compound of formula I or II may be liberated by treatment with acid using a conventional procedure for example using an aqueous mineral acid such as dilute hydrochloric acid.

In addition, Example 29 illustrates how a racemic mixture of compounds of Formula I and II may be isolated by chiral chromatography.

As stated earlier, the compounds described herein are modulators of PPAR activity. Thus, in addition to the structural characteristics outlined above, preferred compounds to be used with the methods according to the present invention are also PPAR agonists, PPAR antagonist or PPAR partial agonists, preferably PPAR partial agonists. Methods for determining functionalities as PPAR agonist/antagonist/partial agonist are described herein below in the section "Functionalities of compounds"

### Functionalities of compounds

Compounds capable of modulating the activity of PPAR (herein also referred to as PPAR ligands) can be grouped into three distinct classes: Full agonists, partial agonists/partial antagonists, and full antagonists. Agonists and antagonists are characterized by their binding affinities, dictating potency/EC50/IC50 values, and by the level of activity, which is attained in the presence of saturating levels of the compounds, i.e. efficacy. A partial agonist/partial antagonist is also characterized by its binding affinity, and efficacy. A partial agonist/partial antagonist is unable to fully activate the cognate PPAR and can in a competitive manner displace a full agonist from the receptor and thereby diminish the level of transactivation. Full and partial agonists furthermore may recruit different complements of cofactors, and the nature of the cofactors recruited to a given PPAR subtype may profoundly influence the pattern of genes activated by a given agonist.

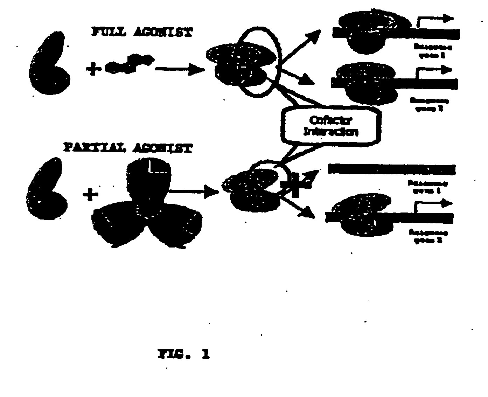

The ligand-binding pockets of the PPARs are large compared with other nuclear receptors, and this may in part explain the large variety of compounds that are able to bind to and activate the PPARs. There is a considerable overlap in ligand recognition between the three PPAR subtypes, and strictly speaking, no subtype specific ligand has yet been identified. However, several natural and synthetic ligands exhibit a great degree of selectivity, and the most selective ligands today differ by more than 3 orders of magnitude with regard to the concentration needed to activate the individual PPAR subtypes. In analogy with agonists for the steroid nuclear receptors, the term selective PPAR modulators (SPPARMs) has been introduced (herein also referred to as "Partial agonists or antagonists"). This class of ligand comprises partial agonists/antagonists that upon binding to the PPAR(s) introduce different conformations leading to recruitment of different sets of coactivators. In principle, a SPPARM would be able to activate only a subset of PPAR target genes thereby possibly promoting specific expression of a desirable set of genes. A model of PPAR activation by a full agonist and a partial agonist is given in Fig. 1. Preferred compounds according to the present invention are partial PPAR agonists.

PPAR modulating activity can be easily determined by any number of methods known in the art or adaptations thereof. For example, PPAR modulating activity may be determined by a transactivation assay. A non-limiting example of a useful transactivation assay for determining PPARgamma modulating activity is described in example 21, and a non-limiting example of a useful transactivation assay for determining PPARdelta modulating activity is described in example 22. Example 21 below illustrates a method where compounds are added to cells transfected with a construct encoding a PPAR-GAL4 (DNA binding domain) fusion protein and a construct encoding a GAL4 dependent reporter construct. It will be apparent to one of ordinary skill in the art that any number of possible constructs can be used, such as using different DNA binding domains to activate transcription or different reporter genes (for example, fluorescent proteins, beta-galactosidase, peroxidase, luciferase, or the like). It will also be apparent to one of ordinary skill in the art that depending on which PPAR activity it is desirable to determine, the construct preferably encodes said PPAR or a ligand binding domain thereof. Upon activation of PPAR (i.e., in the presence of a PPAR agonist or partial agonist), PPAR transactivates the reporter construct, optionally in a quantitative manner.

PPAR modulators may also be identified using a reporter gene comprising a first nucleic acid operably under control of a second nucleic acid comprising at least one PPRE. The first nucleic acid preferably encodes a reporter protein, such as a fluorescent protein, beta-galactosidase, peroxidase, luciferase, or the like. Said reporter construct should be inserted into a cell expressing one or more PPARs, such as PPARgamma and/or delta. PPAR agonists can thus be identified as compounds capable of activating transcription of the first nucleic acid.

According to a specific embodiment of the invention, the preferred compounds are PPAR and/or PPAR LBD agonists or partial agonists. The term "PPAR LBD" refers to the ligand binding domain of PPAR. According to a preferred embodiment, the compounds and compositions of the present invention are PPARgamma and/or PPARgamma LBD agonists. By "agonist" is meant a compound or composition which when combined with an intracellular receptor stimulates or increases a reaction typical for the receptor, e.g., transcription activation activity. In one embodiment, said agonist is a PPARgamma agonist, i.e., a PPAR ligand which potentiates, stimulates, induces or otherwise enhances the transcriptional activity of a PPARgamma receptor, e.g., such as by mimicking a natural physiological ligand for the receptor.

Said PPAR modulating activity may be determined using the transactivation assays described herein above. Suitable standard agonists include rosiglitazone for PPARgamma and (4-[3-(2-Propyl-3-hydroxy-4-acetyl)phenoxy]propyloxyphenoxy-acetic acid (L165041, commercially available) for PPARdelta. Potential agonists that exhibit less than 50% transactivation than a standard agonist may still be useful, in particular for the development of new compounds or active derivatives or as an indicator of the presence of a partial agonist.

According to a preferred embodiment, the compounds and compositions of the present invention are PPAR and/or PPAR LBD partial-agonists, and more particularly, the compounds and compositions of the present invention are PPARgamma and/or PPARgamma LBD partial-agonists. A drug that produces less than the possible maximal effect (i.e. the maximal effect produced by a full agonist, or reference molecule) is called a partial agonist.

For example, the partial agonist property of the compounds and compositions of the present invention can be defined by reference to rosiglitazone (Avandia^{™}, Glaxo-SmithKline) which is a full agonist. This is in particular the case for PPARgamma partial agonists. The partial PPAR agonists. In particular PPARgamma agonists, of the invention will provide similar or better efficacy to a patient undergoing a desired treatment but will have fewer undesirable side effects which would be detrimental to the patient. For example, SN1 (DPD) induces the same level of glucose uptake at 10mM as Avandia at 1mM but fewer side effects are expected as a consequence of lower adipocyte differentiation.

The partial agonist property of the compounds and compositions of the present invention may also be defined by reference to L165041 (commercially available). This is in particular the case for PPARdelta partial agonists. For example, one such transactivation assay, is the transactivation assay described in example 22.

In one embodiment it is preferred that the compounds of the invention are selective for activation of PPAR. In such an embodiment it is preferred that the compound does not significantly activate RxR and/or RxR LBD transactivation, preferably RxR transcription is less than 2 times background levels, such as less than 1.5 times background levels, for example approximately equal to or less than background level. RxR transactivation may be determined by an RxR transactivation assay, for example as described in example 29. Background level is transactivation in the absence of an added ligand.

In one embodiment of the invention, the compounds are PPAR antagonists.By "antagonist" is meant a compound, which when combined with PPAR interferes or decreases a reaction typical for said PPAR, e.g., transcription activation. As a general definition, "PPAR antagonist" designates a PPAR ligand which can inhibit the activity of a corresponding PPAR agonist. More generally, these agonist/antagonist/partial agonist activities may be measured by assays widely known to one skilled in the art, such as, for example, those which are disclosed in WO99/50664 or WO96/41013. An example of a PPAR antagonist is provided in Example 21.

The compounds and compositions of the invention are further characterized by their biological activities when administered to a patient having a condition or disease that is affected by modulation of PPAR activity. Preferred compounds according to the present invention are compounds capable of lowering one or more of the following biological entities in a patient in need thereof: glucose, triglycerides, fatty acids, cholesterol, bile acid, and the like, with better or equivalent efficacy and potency, but with lower toxicity and/or occurrence of fewer undesirable side effects compared to known molecules in the art (e.g., thiazolidinediones). In particular, said compounds preferably lead to less induction of adipocyte differentiation and weight gain. Such compounds may in particular be any of the compounds described in section C. herein above. Useful methods for determining adipocyte differentiation are described in example 25 herein below. More specifically, they present beneficial activities towards insulin resistance (diminished effectiveness of insulin to lower plasma glucose levels) and/or adipogenesis. It has been shown that many compounds that activate PPARgamma (e.g. thiazolidinediones) further induce adipocyte differentiation (i.e., exhibit an adipogenic, or lipogenic, effect) and thus result in body weight increase in treated patients. Therefore, it is highly desirable that the next generation of such compounds show reduced activity and preferably are devoid of such activity. These activities can be appreciated using methods widely used in the art (such as described in example 5), More specifically, these activities are appreciated with reference to a molecule which has already been identified in the art, such as rosiglitazone. According to a preferred embodiment of the invention, preferred compounds display at least about 50%, preferably at least about 60%, more preferably at least about 70%, and even more preferably at least 80%, of the rosiglitazone property regarding insulin resistance, which, for example, may be determined by determining glucose levels in patients suffering from insulin resistance. Ideally, it will be 100% or more. According to another preferred embodiment of the present invention, preferred compounds display less than about 80%, preferably less than about 50%, more preferably less than about 40%, and even more preferably less than about 30%, of the rosiglitazone property towards adipocyte differentiation. Ideally this will be less than 20% of the rosiglitazone property towards adipocyte differentiation. Adipocyte differentation may, for example, be determined as described herein below in example 30. In a very preferred embodiment, the preferred compounds have both above-mentioned properties. Alternatively, the compounds are capable of inducing PPAR activity as determined in a transactivation assay to an extent which is at least 50% that of rosiglitazone and display the above-mentioned property towards adipocyte differentiation.

The *in vivo* occurrence of undesirable side effects such as haemodilution, oedema, adipocyte differentiation, or obesity may be influenced by the cofactor recruitment profile of said compounds, for example using methods described in EP1267171. Thus, in one embodiment of the invention, preferred compounds are compounds which are predicted to have low *in vivo* occurrence of undesirable side effects.

It is widely acknowledged that nuclear receptors, such as PPARgamma, achieve trancriptional activation or repression by binding to cognate sequences in the promoter regions of target genes and by recruiting numerous cofactor complexes whose activities range from chromatin remodeling, histone and cofactor modification, to basic transcription machinery recruitment (Glass, & Rosenfeld, 2000, Genes Dev., 14, 121-141). These cofactors may to a large extent determine the specificity of the action of nuclear receptors and integrate their action in a network of stimuli whose proper orchestration leads to a specific cellular response. Hence, the determination of the multiple partnerships in which each nuclear receptor is engaged, as a function of time and cell type, will lead to a better understanding of the activity of nuclear receptors in transcriptional regulation. For instance, it is known that for certain hormones, such as estrogen, the response to the hormone is determined almost to the same extent by the presence of the respective nuclear hormone receptor, as by the presence of the cofactors, which interact with the receptor. Various PPAR cofactors have been identified. Some cofactors such as p300/CBP (Dowell et al., 1997, J. Biol. Chem. 272, 33435-33433), SRC-1 (Onate et al., 1995, Science 270, 1354-1357), TIF2 (GRIP-2 ; Chakravarti et al., 1996, Nature, 383, 99-103), SRA (Lanz et al., 1999, Cell, 97, 17-27), AIB-1 (Anzick et al., 1997, Science, 277, 965-968), TRAP220/DRIP205 (i.e. PBP ; Zhu et al., 1997, J. Biol. Chem. 272, 25500-25506 ; Rachez et al., 1999, Nature, 398, 824-828), PGC-1 (Puigserver et al., 1998, Cell 92, 829-839), PRIP (Zhu et al., 2000, J Biol Chem 275, 13510-13516), PGC-2 (Castillo et al., 1999, Embo J , 18, 3676-3687), ARA70 (Heinlein et al., 1999, J Biol Chem 274, 16147-16152), RIP140 (Treuter et al., 1998, Mol Endocrinol 12, 864-881), enhance their transcriptional activity, whereas SMRT (Lavinsky et al., 1998, Proc. Natl. Acad. Sci. USA 95, 2920-2925) and N-CoR (Dowell et al., J Biol Chem 274, 15901-15907) repress it. Additionally, it has been shown that members of the PPARgamma cofactor family (e.g. the 160-kDa protein (SRC-1/TIF2/AIB-1), CBP/p300 or TRAP220/DRIP205) interact directly with PPARgamma and potentiate nuclear receptor transactivation function in a ligand-dependent fashion leading to biological action or side effects that can differ according to the ligand used (Adams et al., 1997, J. Clin. Invest., 100, 3149-3153). Kodera et al, (2000, J Biol Chem., 275, 33201- 33204) have examined whether interactions between PPARgamma and known cofactors were induced to the same extent by different classes of PPARgamma ligands (natural and synthetic) and concluded that the overall structure of PPARgamma and cofactors complexes may be different according to the ligands involved, resulting in the activation of a particular set of target gene promoters that exert different biological actions.

PPAR partial agonists will in general have a particular coactivator recruitment profile, thus compounds with particular coactivator recruitment profiles are preferred. Thus, according to special embodiments, the compounds and compositions of the present invention are furthermore characterized by a restricted cofactor(s) recruitment pattern. In preferred embodiments, said pattern results in distinct effects on the regulation of the transcriptional activity of said nuclear receptors allowing a very finely tuned regulation which results in the activation of specific metabolic processes as well as the elimination of unwanted side effects. In more specific embodiments, the compounds and compositions of the present invention are furthermore able to inhibit the interaction of PPAR receptor, more preferably PPAR receptor LBD with cofactor TIF2. According to another embodiment, the compounds and compositions of the invention are additionally able to enhance the interaction of PPAR receptor, more preferably PPAR receptor LBD, with cofactor SRC-1. Preferably, said PPAR receptor is PPARgamma receptor.

Methods for measuring inhibition and/or enhancement of cofactor recruitment by ligands are detailed in EP1267171.

In a preferred embodiment, the compounds of the invention when bound to PPARgamma will allow recruitment of SRC1 to the LBD with an EC50 which is at least one log greater than the one for TIF2, with at least 2 log being preferred.

In one embodiment of the invention, preferred compounds due to their agonistic, particularly partial agonistic, or antagonistic property towards natural physiological ligands of the PPAR receptors, especially those of the PPARgamma receptor, are capable of serving as pharmaceuticals for controlling the biological effects of PPAR mediated transcriptional control and the attendant physiological effects produced thereby. More specifically, they can modulate a cellular physiology to reduce an associated pathology or provide or enhance prophylaxis.

In yet another embodiment of the invention, preferred compounds are compounds which are agonists (or preferably partial agonists) of more than one PPAR, for example of both PPARgamma and PPARdelta. Such agonists may be identified by transactivation assays for PPARgamma and PPARdelta, respectively, for example as described herein. Non-limiting useful methods for determining PPARgamma and PPARdelta activity are described herein below in examples 26 and 27, respectively.

### Clinical conditions

The present invention relates to 2,4-diphenyl-1,3-dioxanes for use in methods of treatment of clinical conditions comprising administration of above-mentioned compounds, as well as to uses of said compounds for preparation of a medicament for treatment of a clinical condition.

Modulators of PPAR activity may be employed in weight control. Thus, the clinical condition may in one embodiment be an eating disorder such as anorexia nervosa (also abbreviated "anorexia" herein) or bulimia. The compounds disclosed herein above may also be employed in methods for increasing or decreasing body weight, in particular for decreasing body weight. The clinical condition may thus be obesity. Adiposity is an excessive build-up of fatty tissue. Recent investigations have shown that PPAR in particular PPARgamma plays a central role in gene expression and differentiation of adipocytes. PPARgamma subtypes are involved in the activation of adipocyte differentiation, but play a less important role in the stimulation of peroxisome proliferation in the liver. Activation of PPARgamma typically contributes to adipocyte differentiation by activating the adipocyte-specific gene expression (Lehmann, Moore, Smith-Oliver, Wilkison, Willson, Kliewer, J. Biol. Chem., 270:12953-12956, 1995). Thus, a PPAR agonist can be used to gain fatty tissue. PPAR partial agonists may be selected for properties useful in treating excessive build-up of fatty tissue. tissue.

In one preferred embodiment, the invention relates to the above-mentioned compounds for use in methods for treating insulin resistance by administering any of the compounds described herein above to an individual in need thereof. The invention also relates to use of any of said compounds for preparation of a medicament for the treatment of insulin resistance. In addition, the invention relates to methods for increasing insulin sensitivity by administration of said compounds, as well as to use of said compounds for the preparation of a medicament for increasing insulin sensitivity. Acute and transient disorders in insulin sensitivity, such as those that may occur following trauma, surgery, or myocardial infarction, may be treated as taught herein.

Insulin resistance is involved in a number of clinical conditions. Insulin resistance is manifested by the diminished ability of insulin to exert its biological action across a broad range of concentrations. During early stages of insulin resistance, the body secretes abnormally high amounts of insulin to compensate for this defect. Even though blood insulin levels are chronically high, the impaired metabolic response of active muscle cells to insulin make them unable to take up glucose effectively. It is now increasingly being recognized that insulin resistance and resulting hyperinsulinemia may contribute to several clinical conditions, for example to the metabolic syndrome (also designated syndrome X). The metabolic syndrome is characterized by a first insulin-resistant stage which causes hyperinsulinemia, dyslipidemia and reduced glucose tolerance. Patients with the metabolic syndrome have been shown to be at an increased risk of developing cardiovascular disease and/or type II diabetes.

A patient is said to suffer from the metabolic syndrome when at least three of the following criteria applies:
- Central/abdominal obesity as measured by waist circumference (greater than 102cm in men and greater than 94cm in women)
- Fasting triglycerides greater than or equal to 150 mg/dL (1.69 mmol/L)
- HDL cholesterol [Men - Less than 40 mg/dL (1.04 mmol/L); Women - Less than 50 mg/ dL (1.29 mmol/L)]
- Blood pressure greater than or equal to 130/85 mm Hg
- Fasting glucose greater than or equal to 110 mg/dL (6.1 mmol/L)

Insulin resistance also has a negative effect on lipid production, contributing to increasing VLDL (very low-density lipoprotein), LDL (low-density lipoprotein), and triglyceride levels in the bloodstream and decreasing HDL (high-density lipoprotein). This can lead to fatty plaque deposits in the arteries which, over time, can lead to atherosclerosis. Thus, the clinical condition according to the present invention may be hyperlipidemia, such as familial hyperlipidemia. Preferably, hyperlipidemia is characterised by hypercholesterolemia and/or hypertriglyceridemia. The clinical condition may also include dyslipidemia and diabetic dyslipidemia. The compounds included herein may also be utilized to lower serum triglyceride levels or raise the plasma level of HDL.

Insulin resistance may lead to excessive insulin and glucose levels in the blood. Excess insulin may increase sodium retention by the kidneys, thus the methods of the invention may be employed for decreasing sodium retention by the kidneys. Elevated glucose levels may damage blood vessels and kidneys. Thus, the methods of the invention may be employed to prevent damage to blood vessels and kidneys.

In another embodiment of the invention, the clinical condition may also be renal diseases, including glomerulonephritis, glomerulosclerosis, nephritic syndrome, and hypertensive nephrosclerosis.

It will be apparent to the artisan that other PPAR subtypes play an important role in disease. For example, PPARdelta has been associated with lipid metabolism disorders and wound healing, in particular epidermal wound healing (Soon Tan et all, 2004, Expert Opinion in Molecular Targets, 39). Thus, the clinical condition may also be wound healing, including epidermal wound healing.

Insulin sensitizers, e.g., glitazones, have been used to treat insulin resistance, but while enhancing insulin sensitivity, they also increase rather than decrease body weight. Obesity and physical inactivity aggravate insulin resistance. Glitazones, (thiazolidinediones or TZDs) act by improving insulin sensitivity in adipose tissue, liver, and muscle. Treatments with said agents have been tested in several animal models of diabetes and resulted in complete correction of the elevated plasma levels of glucose, triglycerides, and nonesterified free fatty acids without any occurrence of hypoglycemic reactions (Cheng Lai and Levine, 2000, Heart Dis., 2,326-333). Examples of these thiazolidinediones are rosiglitazone, pioglitazone and troglitazone. However, while offering attractive therapeutic effects, these compounds suffer from numerous serious undesirable side effects including hemodilution (including oedema), liver toxicity, body weight increase (including body fat increase from increased adipocyte differentiation, plasma volume increase, and cardiac hypertrophy), modest but significant LDL-cholesterol increase and anaemia (for a review, see Lebovitz, 2002, Diabetes Metab. Res. Rev, 18, Suppl 2, S23-9). Indeed a number of available treatments for diabetes are associated with weight gain, a problem of high significance for the long-term management of the disease. Hence, there is much need for alternative, more effective therapeutic agents that can be used in the management of obesity, diabetes and the commonly associated disorders such as cardiovascular and hepatic disease.

Thus, in one embodiment, the invention relates to simultaneous treatment and/or prevention of obesity and diabetes by administering the compounds of the invention to an individual:
i. suffering from obesity and diabetes, or
ii. at risk of acquiring diabetes, and of getting obese, or
iii. suffering from obesity and at risk of acquiring diabetes, or
iv. suffering from diabetes and at risk of getting obese.

The invention also relates to use of the compounds of the invention for preparation of a medicament for the simultaneous treatment and/or prevention of obesity and diabetes. The compounds may be any of the compounds described hereinabove. Within this embodiment, diabetes is preferably diabetes type II. Said individual at risk of acquiring diabetes may, for example, be an individual suffering from the metabolic syndrome described herein above. Said individual at risk of getting obese, may, for example, be an individual under medical treatment with an anti-diabetic compound having the side-effect of weight gain.

The invention also relates to use of any of the specific compounds described above for the preparation of a medicament for treatment or prevention of a clinical condition. The clinical condition may be selected from the group consisting of the metabolic syndrome, dislipidemia, obesity, diabetes mellitus, insulin resistance or any of the conditions related to insulin resistance described above, and wound healing.

Cardiovascular diseases may, for example, be atherogenesis, atherosclerosis or atherosclerotic disorders, vascular restinosi, cardiomyopathy, or myocardial fibrosis, or any of the cardiovascular diseases mentioned above.

Diabetes mellitus refers to a disease process derived from multiple causative factors and characterized by elevated levels of glucose in blood, or hyperglycemia. Uncontrolled hyperglycemia is associated with increased and premature morbidity and mortality. At least two types of diabetes mellitus have been identified : (i) Type I diabetes, or Insulin Dependent Diabetes Mellitus (IDDM), which is the result of a complete lack of insulin, the hormone that regulates glucose utilization under normal physiological conditions, and (ii) the Type II diabetes, or Non Insulin Dependent Diabetes Mellitus (NIDDM). NIDDM is a complex disease derived from multiple causative factors, which can be addressed in some cases by increasing circulating insulin levels.

### Pharmaceutical formulations and methods of administration

In accordance with the methods and compositions of the present invention, one or more of the compounds described herein may be administered to a mammal in a variety of forms depending on the selected route of administration, as will be understood by those skilled in the art. The compositions of the invention may be administered orally or parenterally, the latter route including intravenous and subcutaneous administration. Parenteral administration may be by continuous infusion over a selected period of time.

Forms for injectable use include sterile aqueous solutions or dispersion and sterile powders for the extemporaneous preparation of sterile injectable solutions or dispersions. In all cases, the form must be sterile and must be fluid to the extent that easy syringability exists.

For ease of administration by the patient, oral or other non-invasive modes of administration are preferred, e.g. patches, suppositories and the like. The compounds may be orally administered with an inert diluent or with an assimilable edible carrier, or it may be enclosed in hard or soft shell gelatin capsules, compressed into tablets or incorporated directly with the food of the diet. For oral therapeutic administration, a compound may be incorporated with excipient and used in the form in ingestible tablets, buccal tablets, troches, capsules, elixirs, suspensions, syrups, wafers and the like.

Compositions containing one or more compounds of the present invention can also be administered in a solution or emulsion contained within phospholipid vesicles called liposomes. The liposomes may be unilamellar or multilamellar and are formed of constituents selected from phosphatidylcholine, dipalmitoylphosphatidylcholine, cholesterol, phosphatidylethanolamtine, phosphatidylserine, dimyristoylphosphatidylcholine and combinations thereof. The multilamellar liposomes comprise multilamellar vesicles of similar composition to unilamellar vesicles, but are prepared so as to result in a plurality of compartments in which the compounds in solution or emulsion is entrapped. Additionally, other adjuvants and modifiers may be included in the liposomal formulation such as polyethyleneglycol, or other materials.

The liposomes containing compositions may also have modifications such as having antibodies immobilized on the surface of the liposome in order to target their delivery.

In one embodiment of the present invention is a pharmaceutical composition for administration to subjects in a biologically compatible form suitable for administration *in vivo* for treating one of the clinical conditions described above in the section "Clinical conditions," said method comprising a safe and effective amount of a compound alone, or in combination with other agents and/or pharmaceutical carriers. For example, the compounds of the invention may be used to treat insulin resistance and/or diabetes in combination with an agent effective against dislipidemia, such as a drug of the fibrate class, e.g., Bezafibrate. The examples of some other agents are insulin sensitizers, PPARγ agonists, glitazones, troglitazone, pioglitazone, englitazone, MCC-555, BRL 49653, biguanides, metformin, phenformin, insulin, insulin minetics, sufonylureas, tolbutamide, glipizide, alpha-glucosidase inhibitors, acarbose, cholesterol lowering agent, HMG-CoA reductase inhibitors, lovastatin, simvastatin, pravastatin, fluvastatin, atrovastatin, rivastatin, other statins, sequestrates, cholestyramine, colestipol, dialkylaminoalkyl derivatives of a cross-linked dextran, nicotinyl alcohol, nicotinic acid: a nicotinic acid salt, PPARalpha agonists, fenofibric acid derivatives, gemfibrozil, clofibrate, fenofibrate, inhibitors of cholesterol absorption, beta-sitosterol, acryl CoA:cholesterol acyltransferase inhibitors, melinamide, probucol, PPARdelta agonists, antiobesity compounds, fenfluramine, dexfenfluramine, phentiramine, sulbitramine, orlistat, neuropeptide Y5 inhibitors, β₃ adrenergic receptor agonists, and ileal bile acid transporter inhibitors.

The composition may be administered to any living organism in need of such treatment including humans and animals as the composition has efficacy *in vivo.* By safe and effective, as used herein, is meant providing sufficient potency in order to decrease, prevent, ameliorate, or treat the disease affecting the subject while avoiding serious side effects. A safe and effective amount will vary depending on the age of the subject, the physical condition of the subject being treated, the severity of the disorder, the duration of treatment and the nature of any concurrent therapy, and its determination is within the skill of the ordinary physician. The compositions are formulated and administered in the same general manner as described herein. The compounds of the present invention may be used effectively alone or in combination with one or more additional active agents. Combination therapy includes administration of a single pharmaceutical dosage composition, which contains a compound of the present invention and one or more additional active agents, as well as administration of a compound of the present invention and each active agent in its own separate pharmaceutical dosage. For example, a compound of the present invention and an insulin secretogogue such as sulfonylureas, thiazolidinediones, biguanides, meglitinides, insulin or α-glucosidase inhibitors can be administered to the patient together in a single oral dosage composition such as a capsule or tablet, or each agent administered in separate oral dosages. Where separate dosages are used, a compound of the present invention and one or more additional active agents can be administered at essentially the same time, i.e., concurrently or at separately staggered times, i.e., sequentially; combination therapy is understood to include all these regimens.

A therapeutically active amount of a pharmaceutical composition of the present invention may also vary according to factors such as the disease state, age, sex, and weight of the subject and the ability of a compound to elicit a desired response in the subject. Dosage regimens may be adjusted to provide the optimum therapeutic response. For example, several divided doses may be administered daily or the dose may be proportionally reduced as indicated by the exigencies of the therapeutic situation.

A dose of around 4mg/kg is likely a suitable initial dosage for a mammal and this dosage may be adjusted as required to provide a safe and effective amount. Thus, the dosage will initially typically be 0.1 to 20 mg/kg, preferably 0.5 to 10mg/kg, more preferably 1 to 5mg/kg.

By pharmaceutically acceptable carrier as used herein is meant one or more compatible solid or liquid delivery systems have innocuous physiological reactions when administered to a subject. Some examples include but are not limited to starches, sugars, cellulose and its derivatives, powdered tragacanth, malt, gelatin, collagen, talc, stearic acids, magnesium stearate, calcium sulfate, vegetable oils, polyols, agar, alginic acids, pyrogen free water, isotonic saline, phosphate buffer, and other suitable non-toxic substances used in pharmaceutical formulations. Other excipients such as wetting agents and lubricants, tableting agents, stabilizers, anti-oxidants, and preservatives are also contemplated.

The compositions described herein can be prepared by known methods for the preparation of pharmaceutically acceptable compositions which can be administered to subjects, such that an effective quantity of the compounds or analogs is combined in a mixture with a pharmaceutical acceptable carrier. Suitable carriers are described for example in Remington's Pharmaceutical Sciences (Mack Publishing Company, Easton, PA, USA, 1985). On this basis the compositions include, albeit not exclusively, solutions of the compounds in association with one or more pharmaceutical acceptable vehicles or diluents, and contained in buffered solutions with a suitable pH and iso-osmotic with the physiological fluids.

The following examples describe specific aspects of the invention to illustrate the invention and should not be construed as limiting the invention, as the examples merely provide specific methodology useful in the understanding and practice of the invention.

### Examples

### Example 1 Synthesis of 4-(Z)-6-(2-o-chlorophenyl-4-o-hydroxyphenyl-1,3-dioxan-cis-5-yl) hexenoic acid

This example describes the synthesis of 4-(Z)-6-(2-o-chlorophenyl-4-o-hydroxyphenyl-1,3-dioxan-cis-5-yl) hex-enoic acid, also referred to herein as DPD, according to Scheme 2 (SN1 in Table II).

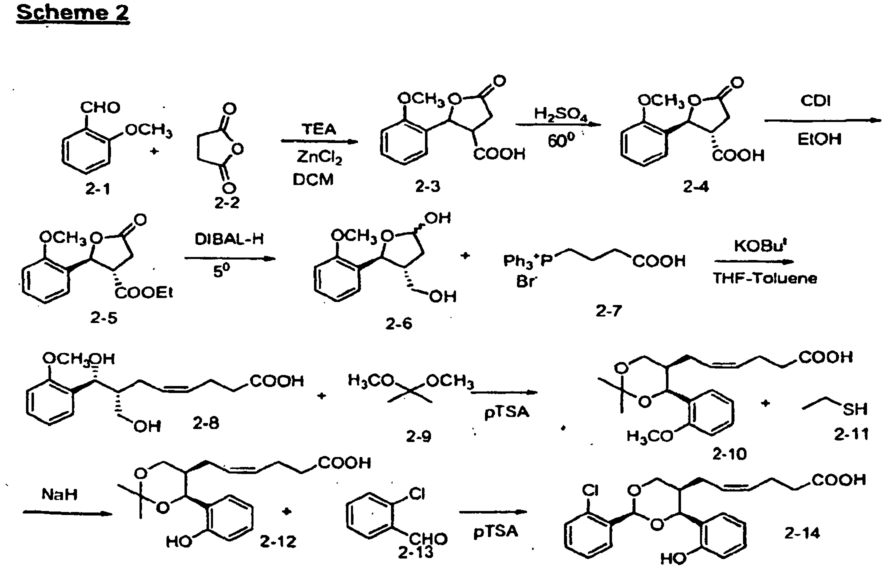

**Synthesis of 2-methoxy-paraconic acid (2-3):** A 20 L double-jacketed glass reactor was charged with 260g o-methoxybenzaldehyde, 286g succinic anhydride, 572g anhydrous zinc chloride and 2600 mL anhydrous DCM . The mixture was stirred and cooled to 2° C. An amount of 533 mL triethylamine was added over a period of 30 min. The mixture was then allowed to stir at ambient temperature for 24h. An amount of 1690 mL 2M HCl was added, followed by 2600 mL ethyl acetate. The mixture was stirred vigorously for 5 min. The aqueous phase was extracted with 2000 mL ethyl acetate. The combined organic extracts were washed with 650 mL saturated brine, followed by a wash with 3 x 2600 mL saturated sodium bicarbonate. The combined aqueous extracts were then washed with ethyl acetate. The aqueous extracts were acidified to pH 2 using concentrated HCl. A yellow oil separated. The mixture was extracted twice using 2000 mL ethyl acetate. The organic phase was washed four times with 1000 mL brine and evaporated on a Buchi R220 rotavap employing a heating bath temperature of 45° C. To the remaining residue were added 4000 mL toluene. The mixture was heated to 110° C. 1 L of toluene was distilled off. The remainder was allowed to cool to room temperature, and was left standing for 48h during which pure 2-methoxy-paraconic acid crystallized. The crystalline material was collected, filtered and dried in vacuo at 45° C in a vacuum oven until constant weight.
**Yield:** 220g (49%). Cis/trans ratio: 46/54

**Conversion of *cis*- and *trans* racemic methoxy-paraconic acid to all-*trans*-2-methoxy para conic acid (2-4):** 1020g methoxyparaconic acid was added to a mixture of 1729 mL concentrated sulfuric acid and 2570 mL water. The mixture was allowed to stir at room temperature for 18h. A cis/trans ratio of 33:64 was obtained. The mixture was then heated to 60° C for 2.5h. A *cis*/*trans* ratio of 11:89 was obtained (analysis by HPLC). The mixture was then allowed to cool to room temperature, and subsequently filtered. The solid material was redissolved in ethyl acetate, and washed with water and brine. The organic layer was dried over MgSO₄ and evaporated. A *cis*/*trans* ratio of 6:94 was observed. The solid material was recrystallized from hot toluene. The obtained crystalline material was dried in vacuo at 40° C for 48h.
**Yield:** 855g (84%). *cis*/*trans* ratio: 8/92. Melting point: 132-133°C
NMR (CDCl₃. 300 MHz): 2.9 (2H, d), 3.4 (1H, m), 3.83 (3H, s), 5.85 (1H, d), 6.8-7.4 (4H, m)

**Esterification of methoxy-paraconic acid,** Racemate **(2-5):** 193g of methoxy-paraconic acid, were dissolved in 600 mL THF. To the mixture were added 145g CDI (899 mmol, 1.1 eq), and the mixture was stirred for 10 min. An amount of 65 mL absolute ethanol (or methanol to make the methyl ester) was added, and the mixture was stirred till complete (∼ 120 min). The crude reaction mixture was extracted using ethyl acetate and saturated sodium bicarbonate. The organic phase was washed with 0.5N HCl and brine. After evaporation, an amount of 188g of the desired ethyl ester were obtained.

**Reduction of racemic methoxy-paraconic acid, ethyl ester (2-6):** Preparation of racemic lactol: 105g ethyl ester (397 mmol) in 700 mL toluene at 5° C. Added 3 eq. DIBAL-H (1.19 mol, 1.19L 1M solution). Stirred for 60 min at room temperature. Quenched with methanol. Added 2.5 L ethyl acetate. 700 mL water. Extracted aqueous phase with EtOAc. Washed the organic layer with brine. Evaporated, recrystallized oily residue from chloroform/hexanes. The solids were filtered and dried in vacuo.
**Yield:** 53g (237 mmol, 59%).

**Wittig reaction employing racemic lactol-Synthesis of racemic diol (2-8):** An amount of 191g carboxypropyltriphenylphosphonium bromide, 1000 mL anhydrous toluene and 100g potassium t-butoxide were mixed at 80°. C for 30 min. The mixture was cooled to room temperature. An amount of 25g purified racemic lactol (114.5 mmol) pre-dissolved in 180 mL anhydrous THF were slowly added. The reaction was continued for 60 min. The crude reaction mixture was poured into 1500 mL ice-water, 300 mL ethyl acetate were added. The aqueous phase was re-extracted with 300 mL ethyl acetate. The aqueous phase was then acidified with 2N HCl, and extracted 3 times using 300 mL ethyl acetate. The solids that had formed were filtered off. The organic phase was evaporated. To the evaporated residue were added 500 mL diethyl ether. The flask was swirled for 10 min, and the solids were filtered off. The filtrate was extracted 3 times with saturated sodium bicarbonate solution. The aqueous phase was then acidified to pH 4 using 2M HCl. The aqueous phase was then extracted 3 times employing 200 mL of ethyl acetate. The organic phases were combined, dried over MgSO4 and evaporated to yield 45g of material.

**Column chromatography:** Racemic diol was purified over silica gel (35 cm column length, 4 cm diameter). Racemic diol was dissolved in a minimum of ethyl acetate and applied to the column. 1 L of ethyl acetate (60%)/hexanes (40%) was added to a volumetric cylinder. 300 mL of EtOAc/hexanes was taken from the cylinder and added to the column. The remaining 700 mL of EtOAc/hexanes in the cylinder were diluted to 1 L using ethyl acetate. 300 mL of the new EtOAc/hexanes solution were then added to the column and were allowed to pass through the column. The remaining 700 mL of EtOAc /hexanes in the cylinder were again diluted to 1 L using ethyl acetate. 300 mL of the new EtOAc/hexanes solution were then added to the column and were allowed to pass through the column. The remaining 700 L of EtOAc/hexanes in the cylinder were diluted once more to 1 L using ethyl acetate. 300 mL of the new EtOAc/hexanes solution were then added to the column and were allowed to pass through the column. Pure fractions of racemic diol were collected and evaporated to yield 26g of pure racemic diol.
**Yield:** 26g (88.3 mmol, 79%)

**Conversion of racemic diol into racemic acetonide (2-10):** 26g (88 mmol) of purified diol was mixed with 260 mL dimethoxypropane and 26 mg p-TsOH. The mixture was allowed to stir at ambient temperature overnight. Three drops of triethylamine were added, and the mixture was evaporated. To the remaining residue were added 150 mL hexane, and the mixture was stirred overnight. The solids were filtered off and dried to yield 25g (75 mmol) of racemic acetonide.
**Yield:** 85%

**De-methylation of racemic acetonide (2-12):** A suspension of sodium hydride and ethanethiol was prepared by adding 16.7g of ethanethiol to a mixture of 21.5g NaH in 375 mL DMPU. The suspension was heated to 80° C, and allowed to cool to ambient temperature.
15g of racemic acetonide were dissolved in 75 mL DMPU and added to the suspension of EtSH/NaH. The mixture was heated at 130° C for 2h. The reaction mixture was then poured into ice-water and extracted with DCM. The aqueous layer was acidified using 2N HCl, and extracted with ethyl acetate. The organic layer was washed with brine and evaporated to dryness.
**Yield:** 16.5g (crude).

**Preparation of Racemic (2-14):** An amount of 28 mmol de-methylated racemic acetonide was mixed with 15 mL 2-chlorobenzaldehyde, 0.5g of p-TsOH, and 60 mL of toluene. The mixture was stirred for 24h and evaporated. The crude reaction mixture was purified using silica gel chromatography employing a Biotage Horizon® chromatography instrument. The mixture was purified using DCM (19)/methanol(1) to yield 6.5g of a solid after evaporation.
**Yield:** 6.5g (16.7 mmol, 59%)

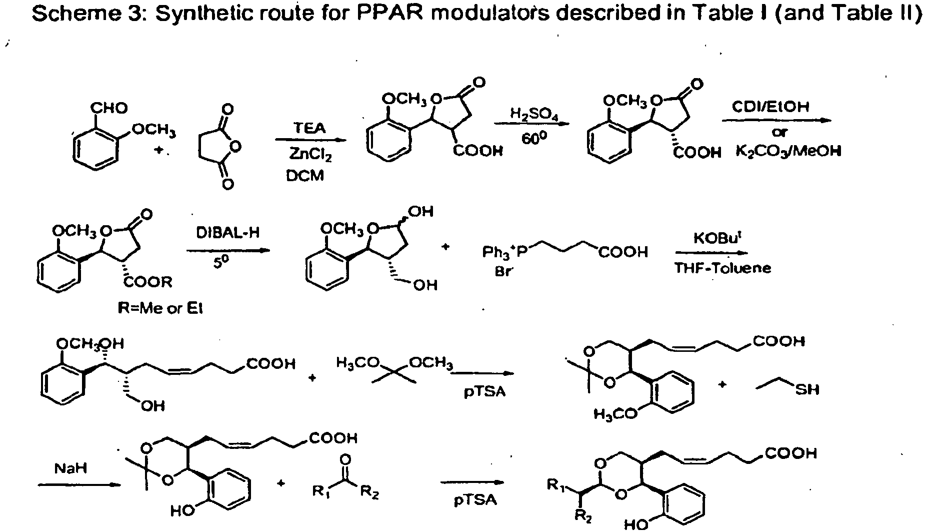

**Table I**

| | R₁ | R₂ |
|---|---|---|
| | 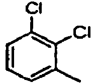 | H |
| | 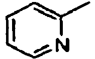 | H |
| | 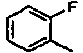 | H |
| | 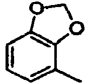 | H |
| | 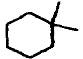 | |
| | 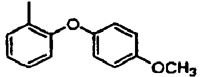 | H |
| | 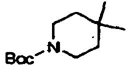 | |
| | 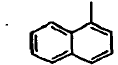 | H |
| | 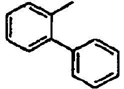 | H |
| | 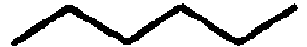 | H |
| | 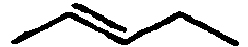 | H |
| | 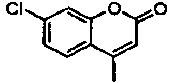 | H |
| | 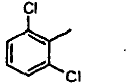 | H |
| | 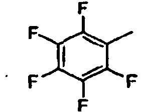 | H |
| | 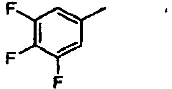 | H |
| | 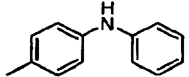 | H |
| | 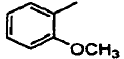 | H |
| | 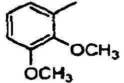 | H |
| | 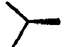 | H |
| | 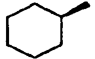 | H |
| | 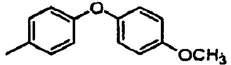 | H |

### Example 2 Synthesis of PPAR modulator 2 (SN2, Table II)

This example describes the synthesis of PPAR modulator 2 (SN2, Table II) according to Scheme 3. An amount of 100 mg (0.31 mmol) racemic acetonide (6-[4-(2-hydroxyphenyl)-2,2-dimethyl-[1,3]dioxan-5-yl]-hex-4-enoic acid) 2-12 as described in Example 1. was mixed with 1 mL toluene and 10 mg p-toluenesulfonic acid. To the mixture was added 2 eq (0.62 mmol, 108 mg) of 2,3-dichlorobenzaldehyde. The mixture was stirred for 24h and evaporated using a nitrogen flow. The crude reaction mixture was purified over a silica gel column, using methanol(1)/DCM(19). Pure fractions were identified by TLC, collected, evaporated and analyzed by HPLC and mass spectrometry.
Mass spectrum (electrospray, negative mode): [M-H]- = 435

### Example 3 Synthesis of PPAR modulator 6 (SN6, Table II)

This example describes the synthesis of PPAR modulator 6 (SN6, Table II) according to Scheme 3. An amount of 100 mg (0.31 mmol) racemic acetonide was mixed with 1 mL toluene and 10 mg p-toluenesulfonic acid. To the mixture was added 2 eq (0.62 mmol, 70 mg) of cyclohexanone. The mixture was stirred for 24h and evaporated using a nitrogen flow. The crude reaction mixture was purified over a silica gel column, using methanol(1)/DCM(19). Pure fractions were identified by TLC, collected, evaporated and analyzed by HPLC and mass spectrometry.
Mass spectrum (electrospray, negative mode): [M-H]- = 359

### Example 4 Synthesis of PPAR modulator 8 (SN8, Table II)

This example describes the synthesis of PPAR modulator 8 (SN8, Table II) according to Scheme 3.

**Preparation of N-Boc 4-oxopiperidine:** An amount of 2g 4-oxopiperidine was dissolved in 20 mL dioxane/water. To the mixture was added 2 eq. Sodium bicarbonate, followed by 1.0 eq. Boc2O. The mixture was stirred for 4h. Ethyl acetate (100 mL) was added. The organic phase was washed twice using 0.2N HCl and brine. The organic phase was then dried over magnesium sulfate and evaporated to yield oil which crystallized. An amount of 100 mg (0.31 mmol) racemic acetonide was mixed with 1mL toluene and 10 mg p-toluenesulfonic acid. To the mixture was added 2 eq. (0.62 mmol) of N-Boc-4-oxopiperidine. The mixture was stirred for 24h and evaporated using a nitrogen flow. The crude reaction mixture was purified over a miniature silica gel column, using methanol(1)/DCM(19). Pure fractions were identified by TLC, collected, evaporated and analyzed by HPLC and mass spectrometry.
Mass spectrum (electrospray, negative mode): [M-H]- = 460

The biological assays referred to hereinbelow were carried out with the reaction product including the Boc-protecting group still present on the spiro-piperidine ring.

### Example 5 Synthesis of PPAR modulator 9 (SN9, Table II)

This example describes the synthesis of PPAR modulator 9 (SN9, Table II) according to Scheme 3.

**Preparation of 1-naphthalenecarboxaldehyde** An amount of 3.87 mL oxalyl chloride (44.24 mmol) was dissolved in 100 mL DCM. The mixture was cooled to -60° C. An amount of 5.6 mL DMSO was added dropwise using a syringe. The mixture was stirred for 15 min. A solution of 5g of naphthalene-1-methanol in 75 mL DCM was added dropwide. The reaction was continued for 1h at -60°C. An amount of 20 mL triethylamine was added. The mixture was allowed to attain 15° C. The mixture was transferred to an extraction funnel, washed with water, 1 M HCl, water and brine. The organic layer was then dried over magnesium sulfate and evaporated. The crude product was sufficiently pure to be used without further purification in the next step.

An amount of 100 mg (0.31 mmol) racemic acetonide (2-12 as described above) prepared according to Example 1 was mixed with 1 mL toluene and 10 mg p-toluenesulfonic acid. To the mixture was added 2 eq. (0.62 mmol, 97 mg) of 1-naphthalenecarboxaldehyde. The mixture was stirred for 24h and evaporated using a nitrogen flow. The crude reaction mixture was purified over a silica gel column, using methanol(1)/DCM(19). Pure fractions were identified by TLC, collected, evaporated and analyzed by HPLC and mass spectrometry.
Mass spectrum (electrospray, negative mode): [M-H]- = 417

### Example 6 Synthesis of PPAR modulator 11 (SN11, Table II)

This example describes the synthesis of PPAR modulator 11 (SN11, Table II) according to Scheme 3.

An amount of 100 mg (0.31 mmol) racemic acetonide (2-12 from Example 1) was mixed with 1 mL toluene and 10 mg p-toluenesulfonic acid. To the mixture was added 2 eq (0.62 mmol, 62 mg) of hexanal. The mixture was stirred for 24h and evaporated using a nitrogen flow. The crude reaction mixture was purified over a silica gel column, using methanol(1)/DCM(19). Pure fractions were identified by TLC, collected, evaporated and analyzed by HPLC and mass spectrometry.
Mass spectrum (electrospray, negative mode): [M-H]- = 361

### Example 7 Synthesis of PPAR modulator 13 (SN13, Table II)

This example describes the synthesis of PPAR modulator 13 (SN13, Table II) according to Scheme 3..

An amount of 100 mg (0.31 mmol) racemic acetonide was mixed with 1 mL toluene and 10 mg p-toluenesulfonic acid. To the mixture was added 2 eq (0.62 mmol, 130 mg) of o-2-oxo-4a,8a-dihydro-2H-chromene-4-carbaldehyde. The mixture was stirred for 24h and evaporated using a nitrogen flow. The crude reaction mixture was purified over a miniature silica gel column, using methanol(1) /DCM(19). Pure fractions were identified by TLC, collected, evaporated and analyzed by HPLC and mass spectrometry.
Mass spectrum (electrospray, negative mode): [M-H]- = 469

### Example 8 Synthesis of PPAR modulator 19 (SN19, Table II)

This example describes the synthesis of PPAR modulator 19 (SN19, Table II) according to Scheme 3.

An amount of 100 mg (0.31 mmol) racemic acetonide was mixed with 1 mL toluene and 10 mg p-toluenesulfonic acid. To the mixture was added 2 eq (0.62 mmol, 103 mg) of 2,3-dimethoxybenzaldehyde. The mixture was stirred for 24h and evaporated using a nitrogen flow. The crude reaction mixture was purified over a silica gel column, using methanol(1)/DCM(19). Pure fractions were identified by TLC, collected, evaporated and analyzed by HPLC and mass spectrometry.
HPLC: retention time (gradient A): 15.23, 15.39 min, 95% (sum of 1:1 mixture of isomers)
Mass spectrum (electrospray, negative mode): [M-H]- = 427.2

**Example 9 Synthesis of PPAR modulator 14 (SN14, Table II)** This example describes the synthesis of PPAR modulator 14 (SN14, Table II) according to Scheme 3. according to Scheme 3.

To a stirred solution of diol (0.08g, 0.27 mmol) in CH2Cl2 (10 mL) at room temperature was added 2,6-dichlorobenzaldehyde (0.07g, 0.40 mmol) and pTSA (3 mg). The reaction mixture was stirred for 8h and quenched with triethylamine (2-3 drops) before concentration in vacuo. The residue was purified by column chromatography on silica gel eluted with hexane/EtOAc (8:2) to give the acetal (100 mg, 45% yield) as colorless oil.
¹HNMR (200 MHz, CDCl₃): δ 7.55 (d, 1H, *J* = 7.8 Hz), 7.37-7.13 (m, 4H), 7.00 (t, 1H, *J* = 7.8 Hz), 6.87 (d, 1H, J = 7.8 Hz), 6.46 (s, 1H), 5.51-5.15 (m, 3H), 4.24-4.14 (m, 2H), 3.84 (s, 3H), 2.99-2.81 (m, 1H), 2.40-2.27 (m, 4H), 1.93-1,87 (bd, 1H, *J* = 10.9 Hz), 1.73-1.67 (bd, 1H, *J* = 10.9 Hz). MS: 450 (M⁺)

### Example 10 Synthesis of PPAR modulator 15 (SN15, Table II)

This example describes the synthesis of PPAR modulator 15 (SN15, Table II) according to Scheme 3. Compound 2-12 from Scheme-2, Example 1 (100 mg, 0.31 mmol) was dissolved in THF (5 mL), and a catalytic amount of pTsOH (5 mg) was added at room temperature. The reaction mixture was kept for stirring at room temperature for 8h. 2,3,4,5,6-Pentafluoro benzaldehyde (158 mg, 0.62 mmol) was added to the reaction mass; once again catalytic amount of pTsOH was added. Reaction conditions were maintained for further 24h. After the completion of reaction, dry Et3N was added to adjust to pH=7. The solvent was removed under vacuum and the resulting crude mixture was purified by column chromatography to obtain final product 15.

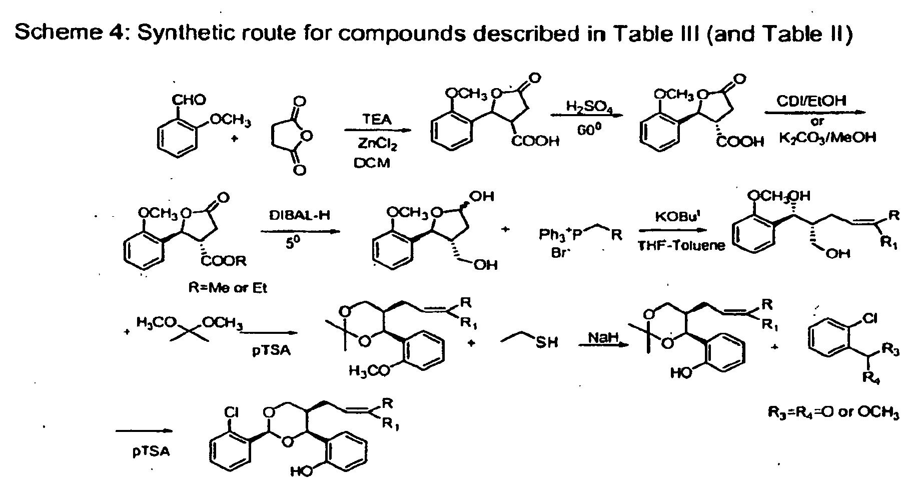

**Table III**

| | R | R₁ |
|---|---|---|
| | 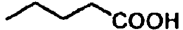 | H |
| | 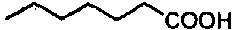 | H |
| | 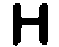 | COOH |
| | 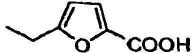 | H |

### Example 10 Synthesis of PPAR modulator 23 (SN23, Table II)

This example the synthesis of PPAR modulator 23 (SN23, Table II) according to Scheme 4.

**Wittig reaction** An amount of 10g (22.56 mmol) BrPPh₃(CH₂)₄CO₂H was mixed with 5.06g (45 mmol) KOtBu in 60 mL dry toluene. The mixture was heated to 80° C for 30 min and allowed to cool to ambient temperature. An amount of 1.26g racemic lactol in 10 mL anhydrous THF was added and the reaction was continued for 2h. Work-up as for preparation of 2-8 Scheme-2.
Yield: 3.3g (crude, used as is in the next step).

**Preparation of acetonide** The diol, obtained in the previous step, was dissolved in 40 mL dimethoxypropane. An amount of 25 mg p-TsOH was added and the reaction was stirred for 24h. A drop of triethylamine was added and the mixture was evaporated. The crude mixture was filtered over a small silica gel column.
**Yield:** 1.6g

**De-methylation of acetonide** An amount of 1.91 mL ethanethiol (25.83 mmol) was mixed with 50 mL DMPU. An amount of 51.7 mmol NaH (2.06g 60% dispersion in oil) was added and the mixture was heated to 80° C for 30 min. The mixture was cooled to ambient temperature. A solution of 1.5g acetonide (4.3 mmol) in 7.5 mL DMPU was added and the mixture stirred for 2h at 125° C. The crude mixture was poured into ice-water, and extracted with 2 x 50 mL DCM. The aqueous phase was acidified with 2N HCl, then extracted with EtOAc, and finally washed with brine. The organic phase was evaporated to yield 1.6g of hydroxy-acetonide.

**Reaction with 2-chlorobenzaldehyde** The hydroxy-acetonide (1.6g, crude) was mixed with 2 mL 2-chlorobenzaldehyde, 8 mL anhydrous toluene, and 50 mg pTsOH. The mixture was stirred for 24h, evaporated, and purified by silica gel column chromatography.
**Mass spectrum** (electrospray, negative mode): [M-H]- = 415

### Example 11 Synthesis of PPAR modulator 17 (SN17, Table II)

This example describes the synthesis of PPAR modulator 17 (SN17, Table II) according to Scheme 4.

An amount of 100 mg (0.31 mmol) racemic acetonide was mixed with 1 mL toluene and 10 mg p-toluenesulfonic acid. To the mixture was added 2 eq (0.62 mmol, 122 mg) of 4-phenylaminobenzaldehyde. The mixture was stirred for 24h and evaporated using a nitrogen flow. The crude reaction mixture was purified over a miniature silica gel column, using methanol(1)/DCM(19). Pure fractions were identified by TLC, collected, evaporated and analyzed by HPLC and mass spectrometry.

### Synthesis of methyl ester of o-methoxyparaconic acid:

a) To a stirred solution of acid (5g, 21.18 mmol) in dry DMF (150 mL) was added K₂CO₃ (29.2g, 211.8 mmol) followed by methyl iodide (6.01g, 42.3 mmol) at 0° C and the reaction mixture was stirred for 5h at room temperature. The reaction mixture was diluted with water (10 mL), CH₂Cl₂ (30 mL) and the organic layer was separated. The aqueous layer was extracted with CH₂Cl₂ (2 x 15 mL) and the combined organic layers were washed with brine (2 x 20 mL), dried over Na₂SO₄ and evaporated in vauo to obtain the ester, which was pure enough to use in further reaction. (Yield: 4.34g, 81.9 %).
b) To a stirred solution of acid 5 (10g, 42.3 mmol) in dry ether (60 mL) was added freshly prepared diazomethane solution (200 mL solution, generated from 10g of nitraso methyl urea) at 0° C and stirred for 30 min at r. t. After complete disappearance of starting material, ether was evaporated to get the ester 6, which was used for the further reaction without purification. (Yield: 9.8g, 98%). Yellow viscous oil
   ¹H NMR (CDCl₃, 200 MHz): δ 7.38-7.22 (2H, m), 6.94-6.82 (m, 2H), 5.79 (d, J= 6.2 Hz, 1H), 3.36-3.24 (m, 1 H), 2.9-2.8 (m, 2H).

### Example 12 Synthesis of PPAR modulator 34 (SN34, Table II)

This example describes the synthesis of PPAR modulator 34 (SN34, Table II).

A mixture of diol 7 (0.4g, 1.36 mmol) and chlorobenzaldehyde dimethylacetal (0.28g, 1.63 mmol) in dry toluene (2 mL) was stirred for overnight in the presence of catalytic p-toluenesulfonic acid (-5 mg) under nitrogen atmosphere. After complete disappearance of the starting material, the reaction mixture was neutralized with solid NaHCO3; the solution was decanted from the reaction mixture and concentrated on rotary evaporator. The crude product was purified by column chromatography to yield benzylidine acetal.
**Yield:** 0.31 g (59%)
¹HNMR (CDCl₃, 200 MHz): δ 7.82 (d, *J*=7.8 Hz, 1H), 7.42 (d, *J*=7.8 Hz, 1H), 7.38-7.2 (m, 3H), 6.87 (t, *J*=7.8 Hz, 1H), 6.82 (d, *J*=8.6 Hz, 1H), 6.05 (s, 1H), 5.72 (d, *J*=15. 6Hz, 1H), 5.45 (d, *J*=2.3 Hz, 1H), 4.21 (t, *J*=15.6 Hz, 2H), 4.1 (q, *J*=7.0 Hz, 2H), 3.83 (s, 3H), 2.68-2.56 (m, 1 H), 1.29 (t, *J*=7.0 Hz, 3H).

To a solution of this compound (0.2g, 0.48 mmol) in THF:H₂O (4 mL, 3:1), was added LiOH.H₂O (0.3g, 7.15 mmol), methanol (0.5 mL) and stirred for overnight at room temperature. The reaction mixture was neutralized with saturated aqueous solution of NaHSO4 and extracted with CH2Cl2 (2 x 10 mL). The combined organic layers were washed with brine (10 mL), evaporated on rotavapour and the crude product was purified by column chromatography.
**Yield:** 95 mg (51%)
¹HNMR (CDCl₃, 200 MHz): δ 7.83 (d, *J*=7.5 Hz, 1H), 7.32 (m, 1H), 7.2 (t, *J*=7.5 Hz, 1H), 6.87 (d. *J*=8.5 Hz, 1H), 6.9 (t, *J*=7.5, 6.6 Hz, 1 H), 6.69 (m, 1H), 6.06 (s, 1 H), 5.67 (d, *J*=16.1 Hz, 1 H), 5.42 (s, 1 H), 4.18 (dd, *J*=11.32 Hz, 2H), 3.84 (s, 3H), 2.58 (m, 1H), 2.09 (m, 2H). MS: 411 (M+Na)⁺

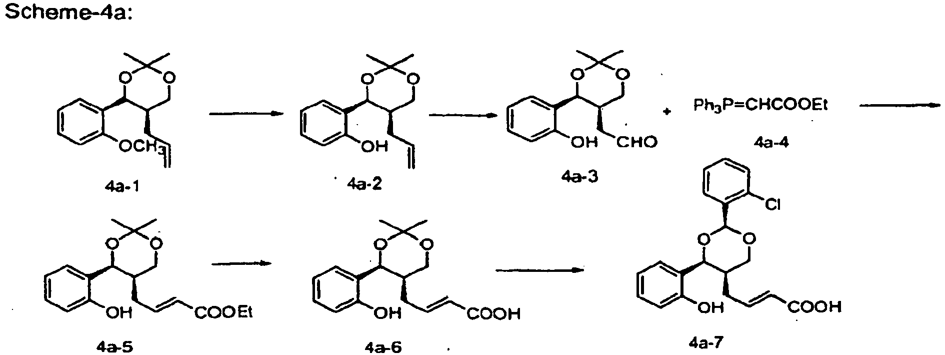

### Example 13 Synthesis of PPAR modulator 25 (SN25, Table II)

This example describes the synthesis of PPAR modulator 25 of Table II.

Ethanethiol (0.7 mL, 9.5 mmol) was added to a stirred suspension of (60% in oil) NaH (0.38g, 9.5 mmol) in dry DMF (8 mL) at 0-5° C and stirred for 20-30 min. Compound 4a-1 (0.25g, 0.95 mmol) in dry DMF (2 mL) was added slowly drop-wise to the above mixture maintaining the temperature. The reaction mass temperature was raised to 120-130° C and maintained for 6-8h. After the completion of reaction, the mass was cooled to 0-5° C and quenched with 1N HCl (1mL) by adjusting the pH 4-5. The compound was extracted with ethyl acetate (3 x 10 mL) and separated the aqueous layer. The combined organic fractions were collected, washed with brine water dried over sodium sulphate (2g), concentrated on vacuum. The crude product was purified by column chromatography to obtain 4a-2 in pure (ethyl acetate:hexane; 2.5:7.5).
**Yield:** 155 mg (65.6 % yield).

Compound 4a-2 (0.15g, 0.6 mmol) was dissolved in a mixture of acetone and water in the ratio of 8:2 (10 mL). To the above mixture, OsO₄ (catalytic amount, 0.05M) and NMO (0.14g, 1.2 mmol) was added and kept for stirring at room temperature for 8-10h. The progress of the reaction was monitored by TLC. After the completion of reaction, it was quenched by saturated Na₂S₂O₅ solution (3 mL). The solvent acetone was removed under vacuum and the compound was extracted with ethyl acetate (3 x 10 mL). The combined organic fractions were collected, washed with brine water dried over sodium sulphate (2g), concentrated on vacuum.

To a solution of the crude product (a diol not depicted in the scheme) in a mixture of THF: H₂O (8:2, 10 mL), NalO₄ (0.27g, 1.8 mmol) was added at room temperature and kept for stirring for 1h. After the completion of reaction, it was quenched by saturated Na₂S₂O₅ solution (3 mL). The compound was extracted with ethyl acetate (3 x 10 mL) and aqueous layer was separated. The combined organic fractions were collected, washed with brine water dried over sodium sulphate (2g), concentrated on vacuum. The crude product 4a-3 was processed for further reaction.

To a solution of compound 4a-3 in dry benzene (10 mL), C2-Wittig ylide (0.15g, 0.42 mmol) was added. The reaction mass was kept for stirring under inert conditions for 2-3h at room temperature. Excess benzene was removed in rotary evaporator and thus resulting crude compound 4a-5 was subjected to purification by column chromatography. (Ethyl acetate: hexane; 0.5:9.5).
**Yield:** 0.11g g (95%)
¹NMR (CDCl₃, 300 MHz): δ 8.1(s, 1H), 7.2-7.1 (t, *J*=6.1 Hz, 1H),6.85-6.6 (m, 4H), 5.8-5.69 (d, *J*=15.7 Hz, 1H), 5.85 (m, 1H), 4.15-4.0 (q, *J*=6.1 Hz, 8.74, 3H), 3.80-3.70 (d, *J*=8.7 Hz, 1H), 2.7-2.55 (m, 1H), 2.15-2.05 (m, 1H), 1.72-1.65 (m, 1H), 1.60=1.40 (m, 6H), 1.25-1.11 (t, *J*=6.1 Hz, 3H), Mass: 343.1 (M⁺+Na)

To a solution of compound 4a-5 (0.11g, 0.34 mmol) in a mixture of THF: H₂O (8:2, 5 mL), LiOH.H₂O (0.1g, 2.4 mmol) was added at room temperature and kept for stirring for 8-10h. After the completion of reaction, it was quenched by saturated NaHSO₄ solution (1 mL) by adjusting the pH to 4-5. The compound was extracted with ethyl acetate (3 x 10 mL) and aqueous layer was separated. The combined organic fractions were collected, washed with brine water dried over sodium sulphate (2g), concentrated on vacuum. The crude product 4a-6 was processed for further reaction.

Compound 4a-6 (85 mg, 0.29 mmol) was dissolved in THF, and a catalytic amount of pTSOH was added at room temperature. The reaction mixture was kept for stirring at room temperature for 6-8h. o-Chlorobenzaldehyde (81 mg, 0.58 mmol) was added to the reaction mass; once again catalytic amount of pTSOH was added. Reaction conditions were maintained for further 5-6h. After the completion of reaction dry Et₃N was added by adjusting the pH=7. The solvent was removed under vacuum and the resulting crude mixture was purified by column chromatography to obtain final product 25 (Table II). (ethyl acetate : hexane;3.5:6.5).
Yield: 35 mg (32 %)

The above compound HPLC purity is 79.2 %, which was further purified by preparative HPLC to obtain the pure compound (98% pure, 15 mg).
¹HNMR (CDCl₃, 300 MHz): δ7.85-7.69(d, *J*=7.1 Hz, 1H), 7.5-7.30(m, 4H), 7.19-7.08( t, *J*=7.1 Hz, 1H), 7.08-7.0( d, *J*=7.1 Hz, 1H), 6.99-6.70 (m, 2H), 6.05(s, 1H), 5.95(s, 1H), 5.9-5.75( d, *J*=15.7 Hz, 1 H), 5.5 (s, 1H), 4.4-4.1 (s, broad, 2H), 2.4-2.1(m, 4H), 2.9-2.7(m, 1 H), 2.35-2.19 (d, *J*=7.1 Hz, 1H), 2.1-1.95 (d, J=7.1 Hz,1H). Mass:374.1 (M⁺+H) HPLC: 98.93% (RT: 4.12)

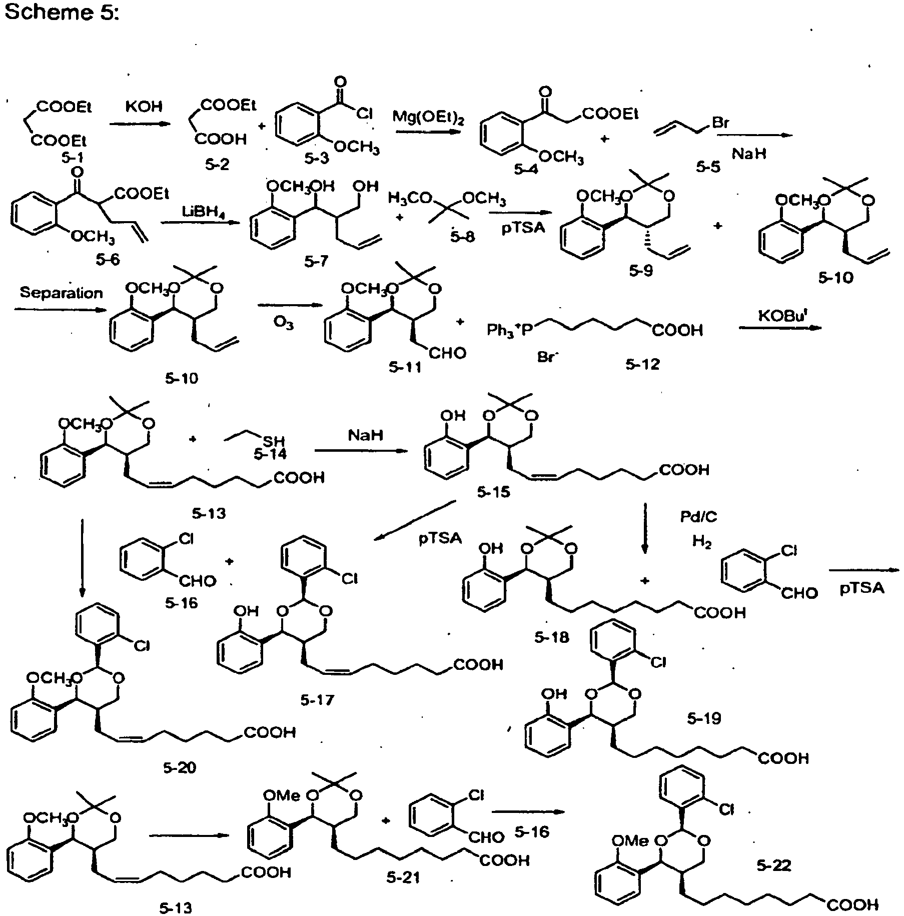

### Example 14 Synthesis of PPAR modulator 37 (SN37, Table II)

This example describes the synthesis of PPAR modulator 37 of Table II according to Scheme 5.

### Preparation of ethyl hydrogenmalonate 5-2 in scheme 5:

To a stirred solution of diethyl malonate (20g, 0.125 moles) in ethanol (100 mL) at r.t, a solution of 85% KOH (7g, 0.125 moles) in ethanol (30 mL) was added with occasional cooling. After 20 min, the mixture was acidified with conc. HCl and filtered. The filter cake (KCI) was washed with ethanol (50 mL). The combined filtrate was concentrated and residual liquid was subjected to column chromatography (hexane-EtOAc: 85:15) to afford 1 (14.7g, 87%). ¹HNMR (CDCl₃): 1.22 (3H, t), 3.39 (2H, s), 4.39- 4.41 (2H, q).

### Preparation of Keto-ester 5-4:

To a suspension of 14.34g of magnesium ethoxide (0.13 moles) in dry THF (100 mL) was added 10.5g of ethyl hydrogenmalonate 1 5-2 (0.08 moles) and refluxed for 90 min. The reaction mixture was cooled to 0° C and a solution of 2-methoxybenzoyl chloride 2 (14.59g; 0.085 moles) in dry THF (25 mL) was added at such a rate that the reaction temperature did not exceed 5° C. The reaction mixture was stirred at r.t for overnight and allowed to stand for two days. A saturated solution of ammonium chloride was added and the reaction mixture was extracted with EtOAc (3 x 50 mL). The combined organic layer was washed with water and dried over anhydrous sodium sulphate. The solvent was evaporated to give the product as oil and was purified by column chromatography (hexane-EtOAc: 95:5) to yield the product (4.33g, 25% yield). The product was confirmed by spectral data. ¹HNMR (CDCl₃): 1.23 (3H, t), 3.85 (5H, s), 4.10- 4.22 (2H, q), 6.90-7.05 (2H, m), 7.42-7.51 (1H, m), 7.69-7.80 (1 H, d).

To a stirred suspension of sodium hydride (60%, 0.9g, 0.08 moles) in dry THF (40 mL), the acetylated ester (4.33g, 0.019 moles) was added drop wise at 0-10 °C and stirred for 15 min. Ally bromide 3 (2.30g, 0.019 moles) was added to the reaction mixture at the r.t. and the reaction mixture was refluxed for 3-4h. The reaction mixture was cooled and ammonium chloride solution was added and extracted with EtOAc (3 x 30 mL). The organic layer was washed with water, dried over anhydrous sodium sulphate and evaporated. The crude product was purified by column chromatography (n- hexane-EtOAc: 95:5) to obtain 4 (2.5g. 50% yield). ¹H-NMR (CDCl₃): 1.15 (3H, t), 2.61-2.72 (2H, m), 3.90 (3H, s), 4.05-4.11 (2H, q), 4.30-4.33 (1H, m), 4.92-5.10 (2H, m), 5.78-5.81 (1H, m), 6.92-7.15 (2H, m), .7.40-7.49 (1 H, m), 7.71- 7.75 (1H, d).

### Preparation of diol (5-7):

To the cooled suspension of lithium borohydride (0.83g, 0.038 moles) in dry THF (15 mL) was added the cooled solution of alkylated keto ester 4 5-4 (2.5g, 0.0095 moles) in dry THF (25 mL) at such a rate that the temperature did not exceed 10°C. Stirring was continued for 4-5h at r.t. and the progress of the reaction was monitored by TLC. The reaction mixture was acidified to pH 2 by the addition of 2N HCl; water was added to the reaction mixture and extracted with EtOAc (3 x 25 mL). The combined organic layer was washed with brine, water and dried over anhydrous sodium sulphate. The solvent was evaporated to yield the *cis* and *trans* reaction mixture of the diol 5 5-7 (2g, 94% yield) and proceeded to the next step with out purification. HPLC: *cis* 77.318%, retention time 20.244 min.; *trans* 22.682%, retention time 22.337 min. (HPLC conditions are mentioned on the chromatogram).

### Preparation of the acetonide 5-10:

A solution of diol 5-7 (2g, 0.009 moles), 2,2-dimethylpropane (15 mL, 0.12 moles), catalytic amount pTSA (10 mg) was stirred at r.t. for 4-5h and the progress of the reaction was monitored by TLC. The reaction mixture was neutralized with Et₃N. Excess DMP was removed under vacuum and the oily *cis-trans* mixture was separated by column chromatography to get 6b 5-10 (0.925g, yield 40%). ¹HNMR (CDCl₃): 1.51 (3H, s), 1.53 (3H, s), 1.62-1.80 (2H, m), 2.25-2.41(1H, m), 3.75-3.85 (1H, m), 3.82 (3H, s), 4.08-4.11 (1H, m), 4.85-4.96 (2H, m), 5.35 (1H,d, *J*=2.3), 5.41-5.62 (1H, m), 6.75-6.81 (1H, d), 6.90-6.97 (1H, m), 7.15-7.25(1H, m), 7.33-7.41(1H, m).

### Preparation 1,3-dioxane aldehyde (Ozonolysis) 5-11:

O₃ was passed through a solution of acetonide 5-10 (0.93g, 0.004 moles) in dry DCM (10 mL) at -78 °C till the permanent blue colour was developed. The reaction mixture was stirred at 0°C till the blue colour was disappeared. A solution of TPP (1.1g, 0.0043 moles) in DCM (5 mL) was added to the colourless solution at 0° C and the reaction mixture was warmed to room temperature and stir for 1h. The progress of the reaction mixture was monitored by TLC. The solvent was removed and the product was purified by flash chromatography to yield the aldehyde 7 (0.5g, yield 54%). ¹H-NMR (CDCl₃): δ 1.50 (3H, s), 1.54 (3H, s), 2.23 (1H, dd), 2.44 (1H, m), 2.75-2.80 (1H, m), 3.65 (1H, dd), 3.81 (3H, s), 4.21-4.22 (1H, dd), 5.39 (1H, d, *J* = 2.2), 6.90 (1H, d), 6.95-6.97 (1H, t), 7.18-7.21 (1H, m), 7.39 -7.40 (1H, d), 9.45 (1H, s). IR (cm⁻¹): 2992, 2933, 2720, 1723, 1595, 1491, 1462, 1379, 1239 and 1197.

### Preparation of Wittig salt 5-12:

A solution of 6-Bromohexanoic acid (3g, 0.0512 moles) and triphenylphosphine 4.8g, 0.018 moles) in dry acetonitrile (50mL) was refluxed for 20-24 h and excess solvent was removed under reduced pressure to afford a color less oil which was triturated with dry benzene and wash in succession with dry benzene and ether (3 times each). During the washing procedure the material crystallized drying at reduced pressure afford Wittig salt as a white microcrystalline powder (3.6g, 55% yield).

### Preparation of 2,4-substituted (1,3-dioxane-5-yl) carboxylic acid (SN37, Table II)] (Wittig product) 5-13:

A solution of sodium hydride (60%, 0.364g, 0.0152 moles) in dry THF (5 mL) was added to a stirred suspension of Wittig salt 8 5-12 (0.95g, 0.002 moles) in dry THF (10 mL) at 0 °C under N₂ atmosphere. The mixture was stirred for 30 min. Then a solution of aldehyde 7 5-11 (0.5g, 0.0019 moles) was added. The reaction was kept under stirring for 36h. After the completion of reaction, water was added and the solvent was removed under reduced pressure. The aqueous solution was washed with ethyl acetate and acidified to pH 2 with 5% HCL and extracted with ethyl acetate. The combined organic extract was washed with saturated brine, dried over anhydrous Na₂SO₄ and evaporated. The oil obtained was purified by flash column chromatography (Hexane: Ethyl acetate -75:25) to afford the Wittig product 9 5-13 (0.2g, 35% yield). 1 H-NMR (CDCl₃): S 1.49 (3H, s), 1.51 (3H, s), 1.40-1.49 (4H, m), 1:55-1.95 (4H, m), 2.22-2.48 (3H, m), 3.70-3.75 (1H, m), 3.80 (3H, s), 4.05-4.15 (1H, m), 5.12-5.42 (2H, m), 5.35 (1H,d, *J*=2.30). 6.75-6.80 (1H, d), 6.92-6.99 (1H, t), 7.21-7.25 (1H, m), 7.40 -7.47 (1H, d). LC/MS: Purity 91% and mass: 385 (M + Na).

### Example 15: Synthesis of PPAR modulator 36 (SN36, Table II)

This example describes the synthesis of PPAR modulator 36 of Table II (2,2-dimethyl-4-(2-hydroxyphenyl)-1,3-dioxane-5-yl-carboxylic acid) (5-15) (deprotection of methoxy group):
Ethanethiol (0.13g, 0.00203 moles) was added at 0 °C under N₂ atmosphere to a stirred suspension of sodium hydride (60%, 1g, 0.004167 moles) in DMPU (5 mL). After 30 min a solution of compound 5-13 (0.00055 moles) in DMPU was added. The mixture was heated at 120 °C for 2-3h, cooled, poured into ice water and the aqueous mixture was washed with DCM . The aqueous layer was acidified to pH 5 with 5% HCl and extracted with ethyl acetate. The combined organic extract was washed with saturated brine dried and evaporated. The oil obtained was purified by flash column chromatography (Hexane: Ethyl acetate - 75:25) to afford Wittig product 5-15 (0.6g, 66% yield). ¹H-NMR (CDCl₃): 1.51 (3H, s), 1.53 (3H, s), 1.22-1.34 (4H, m), 1.52-2.00 (4H, m), 2.21-2.34 (2H, t), 2.53-2.69 (1H, m), 3.79-4.11 (2H, m), 5.15-5.40 (2H, m), 5.38-5.40 (1H,d, *J* =2.40), 6.70-6.95 (3H, m), 7.05- 7.15 (1 H, m), 8.20-8.30 (1 H, br). LC/MS: Purity 99% and mass: 371 (M + Na).

### Example 16 : Synthesis of PPAR modulator 35 (SN35, Table II)

This example describes the synthesis of PPAR modulator 35 of Table II ([2,4,5-cis]-2-o-chlorophenyl-4-o-methoxyphenyl-1,3-dioxan5-yl)octenoic acid - (5-20 scheme 5):
A mixture of 2-chlorobanzaldehyde (50.48 mg, 0.359 mmoles), p-TSA (catalytic, 5 mg) and acetonide compound 5-15 (130 mg, 0.359 mmoles) was stirred in 4 mL of dry toluene for 24h. The solvent was evaporated under reduced pressure and mixture was subjected to column chromatography (hexane-EtOAc: 80:20) to afford product 5-20 (55 mg, 34.3% yield).
   ¹HNMR(CDCl₃, δ): 1.21-2.03 (8H, m); 2.25 (2H, t, *J*=7.554 Hz); 2.28 (1H, m); 3.83 (3H, s); 4.16 (2H, m); 5.13-5.38 (2H, m); 5.41(1H, d, *J*=2.266 Hz); 6.03 (1H, s); 6.81 (1H, d, *J*=7.554 Hz); 6.93 (1H, t, *J*=7.55 Hz); 7.14-7.36 (4H, m); 7.43 (1 H, t, *J*=6.043 Hz); 7.82 (1 H, d, *J*=7.554 Hz). LC-MS: Purity 89.19% (71.05 +18.14, two diastereomers) and mass 462 (M+18).

### Example 17 : Synthesis of PPAR modulator 38 (SN38, Table II)

This example describes the synthesis of PPAR modulator 38 of Table II ([2,4,5-cis]-2-o-chlorophenyl-4-o-methoxyphenyl-1,3-dioxan5-yl)octanoic acid (5-21):
- To the solution of olefin compound 5-13 (150 mg, 0.414 mmol) in 5 mL of dry ethyl acetate, 10 mol% of 10% Pd-C (44 mg) was added carefully. The reaction mixture was allowed to stir under H2 atmosphere for 1.5-2h. After reaction was completed the mixture was filtered through celite and cake (Pd-C) was washed with dry ethyl acetate (2 X 5 mL). The combined filtrate was concentrated and residual liquid was subjected to column chromatography (hexane-EtOAc: 80:20) to afford product 5-21 (SN38) (80 mg, 53.05%yield).
   ¹HNMR (CDCl₃, δ): 1.06-1.60 (12H, m); 1.46 (3H, s); 1.53 (3H, s); 1.67 (1H, m); 2.25 (2H, t, *J*=7.554 Hz); 3.76 (1H, d, *J*=10.009 Hz); 3.81 (3H, s); 4.15 (1H, d, *J*=8.687 Hz); 5.32 (1H, d, *J*=2.455 Hz); 6.77 (1H, d, *J*=7.365 Hz); 6.92 (1 H, t, *J*=7.554 Hz); 7.16 (1H, t, *J*=6.043 Hz); 7.38 (1H, d, *J*=5.854 Hz).

A mixture of 2-chlorobanzaldehyde (34.25 mg, 0.247 mmoles), p-TSA (catalytic, 3 mg) and acetonide compound 12 5-21 (80 mg, 0.34 mmoles) was stirred in 3 mL of dry toluene for 24h. The solvent was evaporated under reduced pressure and mixture was subjected to column chromatography (hexane-EtOAc: 80:20) to afford product 13 5-22 (50 mg, 45.09% yield).
¹HNMR (CDCl₃, δ): 1.08-1.92 (13H, m); 2.26 (2H, t, *J*=7.554Hz); 3.83 (3H, s); 4.20 (2H, m); 5.36 (1H, d, *J*=2.266 Hz); 6.02 (1H, s); 6.81 (1H, d, *J*=7.554 Hz); 6.92 (1H, t, *J*=7.554Hz); 7.15-7.36 (4H, m); 7.42 (1H, t, *J*=7.554 Hz); 7.81 (1H, d, *J*=7.554 Hz).
LC-MS: Purity 87.53% and mass 464 (M+18).

### Example 18 : Synthesis of PPAR modulator 24 (SN24, Table II)

This example describes the synthesis of PPAR modulator 24 of Table II f ([2,4,5-*cis*]-2-o-chlorophenyl-4-o-hydroxyphenyl-1,3-dioxan5-yl)octenoic acid (5-17).

A mixture of 2-chlorobanzaldehyde (48.47 mg, 0.344 mmoles), p-TSA (catalytic, 5 mg) and acetonide compound (120 mg, 0.344 mmoles) was stirred in 4 mL of dry toluene for 24h. The solvent was evaporated under reduced pressure and mixture was subjected to column chromatography (hexane-EtOAc: 80:20) to afford product 5-17 (40 mg, 27% yield).
¹HNMR (CDCl₃, δ): 1.13-2.80 (11H, m); 4.10-4.37 (2H, m); 5.17-5.41 (2H, m); 5.44 (1H, d, *J*=2.340 Hz); 6.00 (1H, s); 6.74-7.74 (8H, m).
LC-MS: Purity is 94.43% (two diastereomers, 80.12+14.31 respectively) and mass 448 (M+18).

### Example 19 Synthesis : Synthesis of PPAR modulator 31 (SN31, Table II)

This example describes the synthesis of PPAR modulator 31 of Table II ([2,4,5-*cis*]-2-o-chlorophenyl-4-o-hydroxyphenyl-1,3-dioxan5-yl)octanoic acid. (5-19)

To the solution of olefin 5-15 (60 mg, 0.172 mmole) in 3 mL of dry ethyl acetate, 10 mol% (19 mg, 0.0172 mmole) of 10% Pd-C was added. The reaction mixture was allowed to stir under H₂ atmosphere for 8h. After reaction was completed the mixture was filtered through celite and cake (Pd-C) was washed with dry ethyl acetate (2 X 5 mL). The combined filtrate was concentrated and residual liquid was subjected to column chromatography (hexane-EtOAc: 80:20) to afford product 5-18 (40 mg, 66.29% yield).
¹HNMR (CDCl₃, δ): 1.05-1.88 (13H, m); 1.54 (3H, s); 1.58 (3H, s); 2.29 (2H, t, *J*=7.031 Hz); 3.86 (1H, d, *J*=11.719 Hz); 4.15 (1H, d, *J*=12.5 Hz); 5.36 (1H, d, *J*=2.344 Hz); 6.82 (3H, m); 7.12 (1H, m); 8.32 (1H, broad).

A mixture of 2-chlorobanzaldehyde (16 mg, 0.114 mmoles), p-TSA (catalytic, 3 mg) and acetonide compound 15 5-18 (40 mg, 0.114 mmoles) was stirred in 3 mL of dry toluene for 24h. The solvent was evaporated under reduced pressure and mixture was subjected to column chromatography (hexane-EtOAc: 80:20) to afford product 16 5-19 (20 mg, 40.28% yield).
¹HNMR (CDCl₃, δ): 1.07-2.35 (13H, m); 2.98 (2H, t, *J*=7.554 Hz); 3.65-4.41 (2H, m); 4.62 (1H, d, *J*=10.575 Hz); 5.90 (1H, s); 6.77-7.66 (8H, m).
LC-MS: Purity 85% and mass 450 (M+18).

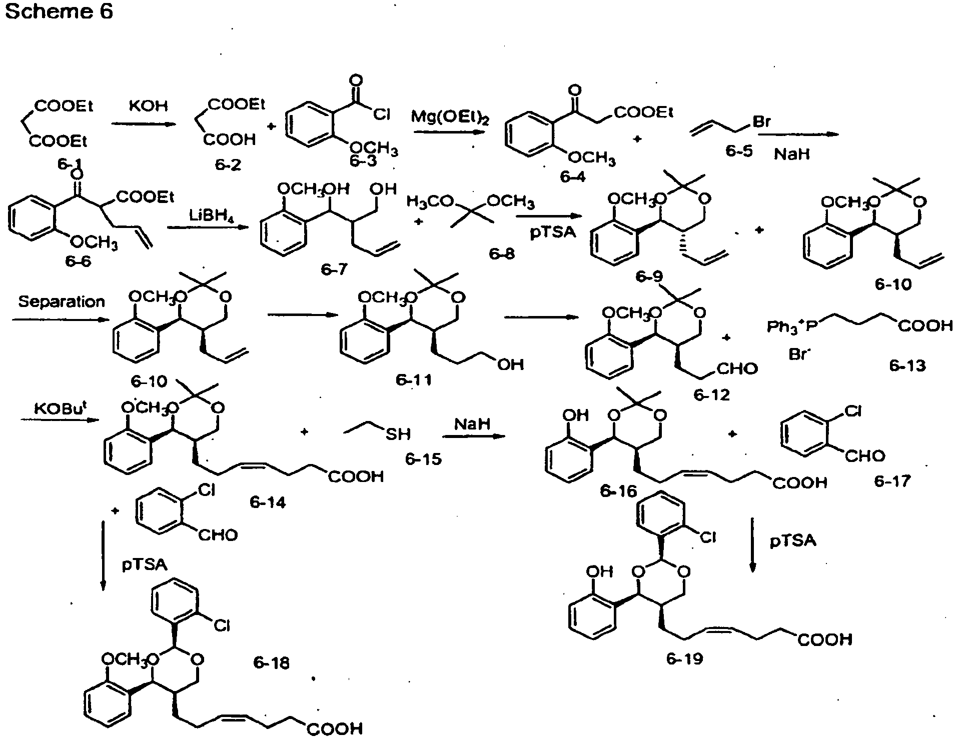

### Example 20 : Synthesis of PPAR modulator 32 (SN32, Table II)

This example describes the synthesis of PPAR modulator 32 of Table II

To a solution of alkene 6-10 (described earlier 5-10) (1.2g, 4.5 mmol) in dry THF (10 mL) was added BH₃.DMS (0.34g, 4.5) at 0°C and the reaction mixture was stirred for 2h at the same temperature. To this mixture was added, 3N NaOH (3 mL) and 30% H₂O₂ (1 mL) at 0°C and stirring continued for another hour at room temperature. The reaction mixture was diluted with water and extracted with ethyl acetate (2 X 30 mL). The combined organic layers were evaporated under vacuum and the crude product was purified by column chromatography (ethyl acetate: hexanes; 2:8).
**Yield:** 0.9 g (70%)
¹H NMR (CDCl₃, 200 MHz): δ 7.51-7.45 (d, J= 6.4 Hz, 1H), 7.3-7.2 (t, *J*= 7.1 Hz, 1H), 7.05-6.95 (t, *J*= 7.1 Hz, 1 H), 6.9-6.82 (d, *J*=7.1 Hz, 1 H), 5.5-5.4 (s 1H), 4.25-4.19 (d, *J*=9.6 Hz, 1H), 3.92-3.78 (m, 4H), 3.53-3.40 (t, *J*=7.1 Hz, 2H), 1.85-1.70 (m, 1 H),1.51 (d, *J*= 11.3 Hz, 6H), 1.32-1.0 (m, 4H).

Preparation of 6-12: IBX (0.525g, 1.87 mmol) was dissolved in dry DMSO (1 mL) and stirred for 10 min at RT and cooled to 0°C. To this, alcohol 6 (0.35g, 1.25 mmol) was added in dry THF (9 mL) under nitrogen atmosphere and stirred for 1.5h at room temperature. The reaction mixture was diluted with ether (10 mL), stirred for 20 min and filtered. The filtrate was washed with water (2 × 10 mL), the ether layer was dried (Na₂SO₄) and concentrated on rotary evaporator. The product was purified by filter column to obtain aldehyde 6-12 (ethylacetate:hexanes; 2:8).
Yield: 300 mg (86.4 % yields).
¹H NMR (CDCl₃, 200 MHz): δ 9.59 (s, 1H), 7.54-7.48 (d, *J*=6.4 Hz, 1H), 7.28-7.20(t, *J*=6.4 Hz, 1H), 7.08-6.95 (t, *J*=6.4 Hz, 1H), 6.90-6.82 (d, *J*=6.4 Hz, 1H), 5.42(s, 1H), 4.26-4.21(d, *J*=9.6 Hz, 1 H), 3.81 (s, 3H), 3.80-3.75 (d, *J*=9.6 Hz, 1H), 2.4-1.7(m, 3H), 1.51 (s, 6H), 1.45-1.40(m, 2H).

Preparation of 6-14: (3-Carboxypropyl)triphenylphosphonium bromide was dried under vacuum at 100°C for 2-3h. To a stirred solution of (3-Carboxypropyl) triphenylphosphonium bromide (1.47g, 3.43 mmol) in dry Toluene (10 mL), Potassium ter-butoxide (0.774g, 6.89 mmol) was added in portions at room temperature under inert conditions. The mixture was heated to 80°C and temperature was maintained for 30-40 mins. Reaction mass was cooled to 50°C and the aldehyde (compound 6-12) (0.3g, 1.07 mmol) in dry THF (2 mL) was added to above mixture drop wise. The progress of the reaction was monitored by TLC. After the completion of reaction, the mass was cooled to 0-5oC and quenched with 1N HCl (1 mL) by adjusting the pH 4-5. The compound was extracted with ethyl acetate (3 X 10 mL) and separated the aqueous layer. The combined organic fractions were collected and dried over sodium sulphate (2g), concentrated on vacuo. The crude product was purified by column chromatography to obtain compound 6-14 in pure (ethyl acetate:hexanes: 2:8).
Yield: 325 mg (86 %).
¹H NMR (CDCl₃, 300 MHz): δ 7.45-7.4 (d, *J*=7.1 Hz, 1H), 7.25-7.10 (t, *J*=7.1, 1H), 6.95-6.85 (t, *J*=7.1 Hz, 1 H), 6.80-6.70 (d, *J*=7.1 Hz, 1H), 5.40-5.30 (s, 1 H), 5.25-5.10 (m, 2H), 4.2-4.1 (d, *J*=14.3 Hz, 1H), 3.85-3.8 (d. *J*=14.3 Hz, 1H), 3.75 (s, 3H), 2.35-2.05 (m, 4H), 1.75-1.5 (m, 3H), 1.50-1.45 (d. *J*=11.4Hz, 6H), 0.85-0.75 (m, 2H).
Mass:371.1 (M⁺+Na).

Preparation of 6-16: Ethanethiol (0.44g, 7.17 mmol) was added to a stirred suspension of 60% NaH (0.287g, 7.17 mmol) in dry DMF (10 mL) at 0-5°C and stirred for 20-30 min. Compound 8 6-14 (0.25g, 0.71 mmol) in dry DMF (2 mL) was added slowly drop wise to the above mixture maintaining the temperature. The reaction mass temperature was raised to 120-130°C and maintained for 6-8h. After the completion of reaction, the mass was cooled to 0-5°C and quenched with 1 N HCl (1 mL) by adjusting the pH 4-5. The compound was extracted with ethyl acetate (3 X 10 mL) and separated the aqueous layer. The combined organic fractions were collected, washed with brine water dried over sodium sulphate (2g), concentrated on vacuum. The crude product was purified by column chromatography to obtain 6-16 in pure (ethyl acetate:hexanes; 2.5:7.5).
Yield: 155 mg (65 %).

Compound 6-16 (130 mg, 0.38 mmol) was dissolved in THF (8 mL), and a catalytic amount of pTsOH was added at room temperature. The reaction mixture was kept for stirring at room temperature for 6-8h. O-chlorobenzaldehyde (109 mg, 0.77 mmol) was added to the reaction mass; once again catalytic amount of pTsOH was added. Reaction conditions were maintained for further 5-6h. After the completion of reaction dry Et₃N was added by adjusting the pH=7. The solvent was removed under vacuum and the resulting crude mixture was purified by column chromatography to obtain final product SN32 (Table II) 6-19. (Ethyl acetate: hexanes; 3.5:6.5).
Yield: 65 mg (40 %).

The HPLC purity after column chromatography is 80.3% with closer impurities. This was further purified by preparative HPLC to obtain 92.4% HPLC pure compound (20 mg obtained).
¹H NMR (CDCl₃, 200 MHz): δ 7.7-7.6 (d, *J*=6.4 Hz, 1H), 7.35-7.20 (m, 3H), 7.10-7.0 (s, 1H), 6.99-6.90 (d, *J*=6.4 Hz, 1H), 6.80-6.60 (m, 2H), 5.95 (s, 1H), 5.85-5.80 (s, 1H), 5.25-5.10 (brs, 2H), 4.27-4.05 (dd, *J*=6.4, 9.6 Hz, 2H), 2.4-2.1 (m, 4H), 2.0-1.65 (m, 3H), 1.29-1.1 (m, 2H).
Mass: 415.1 (M⁺-H)
HPLC purity: 92.48% (column: waters novapak 3.9 × 300 mm; mobile phase: 70% CH₃CN + 30% ammonium acetate buffer; RT: 2.472).

A further analog was synthesized as follows: Compound 6-14 (90 mg, 0.25 mmol) was dissolved in a mixture of THF:0.2N HCl (10 mL, 9:1) and stirred for 2h at room temperature. After completion of the reaction, the mixture was extracted with ethyl acetate (2 x 10 mL), dried over Na₂SO₄ and evaporated on rotary evaporator to obtain 68 mg of crude product, which was utilized for further reaction without purification.

The above crude compound (68 mg, 0.22 mmol) was dissolved in dry THF to this mixture was added 2-chlorobenzaldehyde (61 mg, 0.44 mmol) and catalytic amount of pTSA (~4 mg) at room temperature. The reaction mixture was stirred for 5h at same temperature under nitrogen atmosphere. After disappearance of the starting material, the mixture was neutralized with dry triethyl amine (by adjusting the pH = 7) and the solvent was evaporated. The crude product was purified by column chromatography (25% ethyl acetate in hexanes) to obtain the compound 11.
Yield: 32 mg (34 %).
¹H NMR (CDCl₃, 300 MHz): δ 7.9-7.8 (d, *J*=7.1 Hz, 1H), 7.45-7.15 (m, 5H), 6.95-6.87 (t, *J*= 7.0 Hz, 1 H), 6.85-6.77 (d, *J*=7.2 Hz, 1 H), 6.05 (s, 1H), 5.36 (s, 1H), 5.25 (brs, 2H), 4.2 (2, 2H), 3.85 (s, 3H), 2.35-2.1(m, 4H), 2.05-1.82 (m, 3H), 1.3-1.1(m, 2H).
Mass: 429.1 (M⁺-H)
HPLC purity: 97.84% (column: waters novapak 3.9 × 300 mm; mobile phase: 70% CH₃CN + 30% ammonium acetate buffer (0.05% AcOH; RT: 7.428).

### Example 21 PPARgamma Transactivation Assays

### Cell culture, plasmids and transfections

This is an example of a transactivation assay for determining PPAR gamma modulating activity.

Gal4-PPARgammaLBD (Helledie et al 2000), UASx4-TK luc (Chen and Evans, 1995) and CMV-beta-galactosidase (available commercially, e.g. Clontech) were used in these assays to show PPAR gamma transactivation. The UASx4-TK-luc reporter construct (where UAS refers to "upstream activator sequence") contains four Gal4-responsive elements. The plasmid Gal4-PPARgammaLBD encodes a Gal4-DBD-PPARgamma-LBD fusion protein (i.e. the DNA-binding domain, DBD, of Gal4 fused to the ligand-binding domain, LBD, of PPARgamma) capable of transactivating the UASx4-TK-luc reporter plasmid by binding to the UAS. The CMV-beta-galactosidase plasmid (where CMV is cytomegalovirus) is used for normalization of experimental values.

Mouse embryonic fibroblasts (MEFs) were grown in AmnioMax basal medium (Gibco) supplemented with 7.5 % Amniomax supplement C-100 (Gibco), 7.5% Fetal Bovine Serum (FBS), 2 mM Glutamine, 62.5 microg/ml penicillin and 100 microg/ml Streptomycin (growth medium). Alternatively, ME3 cells (Hansen et al., 1999) were grown in DMEM supplemented with 10% Calf Serum (CS), 62.5 microg/ml penicillin and 100 microg/ml Streptomycin (growth medium). The cells were replated, typically in 24 well plates, so that at the time of transfection the cells are 50-70% confluent.

The cells were transfected with Gal4-PPARgammaLBD (Helledie et al 2000), UASx4-TK luc (Chen and Evans, 1995) and CMV-beta-galactosidase (available commercially, e.g. Clontech) using Lipofectamin Plus (Invitrogen) or Metaffectane (Biontex) according to the manufacturer's instructions. Briefly, per well in a 24 well plate, UASx4TKluc (0.2 microg) Gal4-PPARgammaLBD (or pM-hPPARgamma-LBD; 0.1 microg) and CMV-beta-galactosidase (0.05 microg) in 30 µL DMEM (free of serum and antibiotics) is mixed with 30 microL DMEM (free of serum and antibiotics) containing 1 microL metafectenein. The mixture is incubated at room temperature for 20 min to allow formation of nucleic acid-lipid complexes and then approximately 60 microL is added to each well containing the 50-60% confluent cells. The cells are then incubated at 37°C in a CO₂ incubator for 6 to 12 hours and then the medium is replaced with medium supplemented with antibiotics and the substance of interest (e.g., 4-(Z)-6-(2-o-chlorophenyl-4-o-hydroxyphenyl-1,3-dioxan-cis-5-yl) hexenoic acid, referred to herein as DPD, or rosiglitazone (Avandia) as a positive control, all dissolved in DMSO) or a comparable volume of DMSO (<0.5% of total cell culture volume). DPD is available commercially or can be synthesized according to Example 1. Cells were harvested after 12 -24 hours and luciferase, and beta-galactosidase activities were measured according to standard protocols.

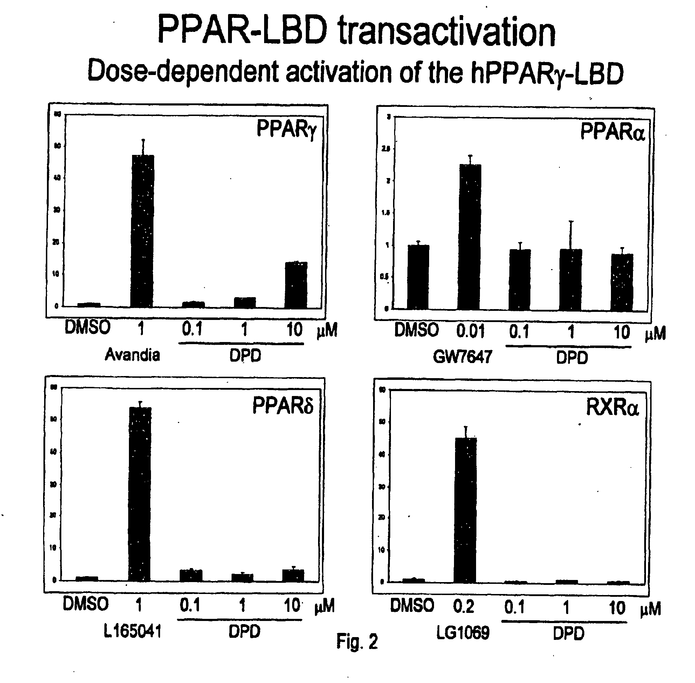

PPAR transactivation was over 40-fold higher with rosiglitazone (a known PPAR gamma agonist) than with DMSO alone, and about 10-fold higher with 10 microM 4-(Z)-6-(2-o-chlorophenyl-4-o-hydroxyphenyl-1,3-dioxan-cis-5-yl) hexenoic acid (see "DPD" in fig. 2). Thus 4-(Z)-6-(2-o-chlorophenyl-4-o-hydroxyphenyl-1,3-dioxan-cis-5-yl) hexenoic acid is a PPAR gamma agonist.

No difference in PPAR gamma transactivating activity was seen between either of the DPD enantiomers purified by chiral HPLC.

Table II summarizes the results obtained with various analogs of DPD (SN1) and compares their activities. "P" represents a similar PPARgamma activating activity at 10 microM compared with 10microM DPD (SN1 in Table II). "P-" represents same level of activity at 30microM tested substance compared with 10microM DPD (ie less potent); "P+" represents level of activity greater than "P" and a level of activity at 3 microM comparable with 10microM DPD (ie more potent); "P++" represents a level of activity at 3 microM above that of 10microM DPD but at 1 microM below that of 10microM DPD; and "P+++" represents a level of activity at 1 microM comparable with 10microM Dud. "-" means that testing was not performed.

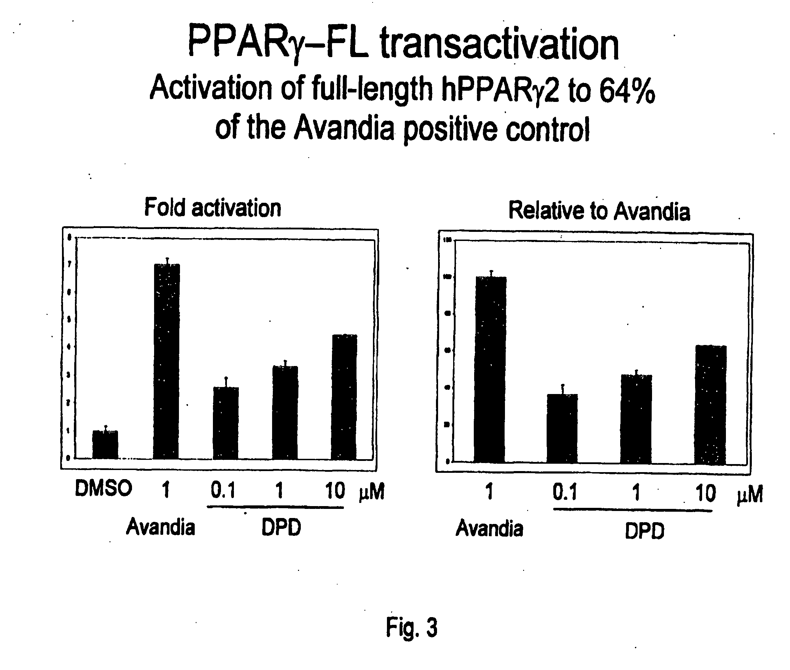

When this assay was carried out essentially as described above but with the full length human PPAR gamma rather than with the PPAR gamma ligand binding domain, activation of full-length hPPARg2 was seen to 64% of the Avandia positive control (Fig 3).

### Example 22 PPARdelta Transactivation Assays

This example describes a transactivation assay for determining PPARdelta modulating activity.

DPD and other ligands are tested for their ability to transactivate PPARdelta essentially as described in Example 1. However, the transactivating construct is mPPARdeltaLBD, where the PPAR delta ligand binding domain replaces that of PPAR gamma and L165041 (commercially available) is used as a selective PPARdelta agonist instead of rosiglitazone. Under the conditions used, L165041 was shown to increase PPAR delta transactivation by over 50-fold, whereas DPD resulted in no or little increase in transactivation (see fig. 2). Therefore DPD shows selectivity for PPAR gamma.

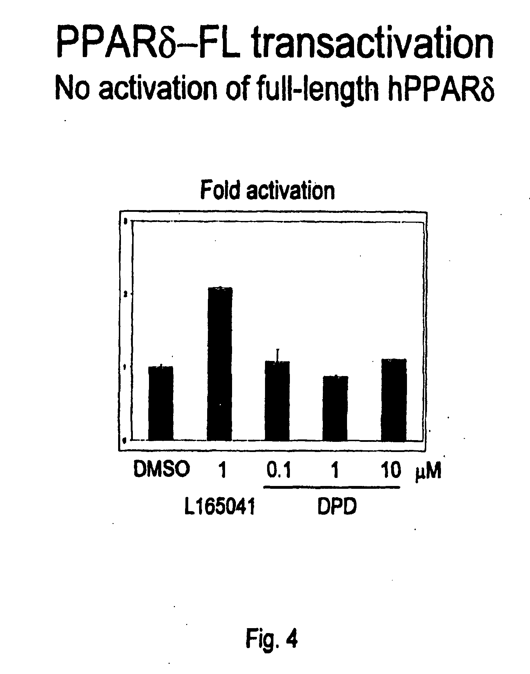

When this assay was carried out essentially as described above but with the full length human PPAR delta rather than with the PPAR deltaa ligand binding domain, activation of full-length hPPARdelta, no activation was seen (Fig 4), confirming the selectivitiy of DPD for PPAR gamma.

### Example 23 PPARalpha Transactivation Assays

This example describes a transactivation assay for determining PPARalpha modulating activity.

DPD and other ligands are tested for their ability to transactivate PPAR alpha essentially as described in Example 1. However, the transactivating construct is mPPARalphaLBD, where the PPAR alpha ligand binding domain replaces that of PPAR gamma and GW7647 (commercially available) is used as a selective PPARalpha agonist instead of rosiglitazone. Under the conditions used, GW7647 was shown to increase PPAR alpha transactivation by over 2-fold, whereas DPD resulted in no transactivation (see fig. 2). Therefore DPD shows selectivity for PPAR gamma.

### Example 24 RXR Transactivation Assays

This example describes a retinoic acid X receptor transactivation assay.

Compounds are tested for their ability to transactivate RXR essentially as described in Example 1. However, the transactivating construct was hRXRαLBD, where the RXR ligand binding domain replaces that of PPAR gamma and {4-[1-(3, 5, 5, 8, 8-pentamethyl-5, 6, 7, 8-tertrahydro-2-naphthyl)ethenyl]benzoic acid} (LG1069, commercially available) is used as a selective RXR agonist instead of rosiglitazone. Under the conditions used, LG1069 is shown to increase RXR transactivation by over 5-fold, whereas DPD resulted in no transactivation (see fig. 2). Therefore DPD again shows selectivity for PPAR gamma.

### Example 25 Adipocyte Differentiation Assays

This example describes an assay for determining adipocyte differention. Compounds are tested to see whether they induce adipocyte differentiation and also to see whether they inhibit adipocyte differention.

### Cell culture and differentiation

MEFs were grown in AmnioMax basal medium (Gibco) supplemented with 7.5 % Amniomax supplement C-100 (Gibco), 7.5% Fetal Bovine Serum (FBS), 2 mM Glutamine, 62.5 µg/ml penicillin and 100 µg/ml Streptomycin (growth medium). At confluence, MEFs were induced to differentiation in growth medium with the addition of 1 microM dexamethasone (Sigma) 0.5 mM isobutylmethylxanthine (Sigma), 5 microg/ml insulin (Sigma) and 10 microM test compound dissolved in DMSO or DMSO alone. Medium was subsequently renewed every 48 hrs with growth media supplemented with 5 microg/ml Insulin and ligand or DMSO.

Briefly, to test compounds as inducers of adipocyte differentiation, 3T3-L1 are grown to confluence in DMEM with 10% Calf serum (CS), typically in 24 well dishes. At 2 days post confluence (day 0), cells are induced to differentiate with DMEM supplemented with 10% fetal bovine serum (FBS), 1 µM dexamethasone and the test compound (01, 1 and 10 microM). BRL49653 (1.0 µM dissolved in 100% Me2SO) is used as a positive control. After 48 h, the cells are re-fed with DMEM containing 10% FBS supplemented with the test compound or the positive control. From day 4, cells are grown in DMEM with 10% FBS and are changed every second day until day 8. At day 8, cells are stained with Oil Red O as decribed below.

To test compounds as inhibitors of adipocyte differentiation, 3T3-L1 cells are grown as above until two-day postconfluence (designated day 0), after which the cells are induced to differentiate with DMEM containing 10% fetal bovine serum (FBS), 1 µM dexamethasone (Sigma), 0.5 mM methylisobutylxanthine (Sigma), 1 µg/ml insulin (Roche Molecular Biochemicals) and the test-compound. Cells induced to differentiate in presence of solvent of the test-compounds are used as positive control. After 48 h, the cells are re-fed with DMEM containing 10% FBS supplemented with the test compound or the positive control. From day 4, cells are grown in DMEM with 10% FBS and are changed every second day until day 8. At day 8, cells are stained with Oil Red O as decribed below.

### Oil Red O staining

Cells cultured as described above are used for Oil Red staining. Dishes are washed in PBS and cells were fixed in 3.7% paraformaldehyde for 1 h and stained with oil red O as described in (Hansen et al 1999). Oil red O solution stock solution is prepared by dissolving 0.5 g of Oil Red O (Sigma) in 100 ml of isopropanol. Oil re O working solution is prepared by diluting a stock solution with water (6:4) followed by filtration.

DPD induced very little red staining in the induction assay. As the red staining is indicative of the presence of adipocytes, it can be inferred that DPD does not induces adipocyte differentiation. However, DPD does not inhibit adipocyte as it showed no significant effect in inhibition of adipocyte differentiation. Table II summarizes results obtained with different analogs of DPD (SN1), "0" representing similar results to DPD, "-1" representing even less adipocyte differentiation, "+1" representing more adipocyte differentiation (both relative to DPD) and "-" meaning not assayed.

An assay carried out essentially as described above but using human pre-adipocytes also demonstrated that DPD induced very little red staining, similar to DMSO.

### Example 26 Identification of Partial Agonists vs. Full Agonists

This example describes an assay for determining partial PPAR agonists, which are particularty desirable as pharmaceuticals.

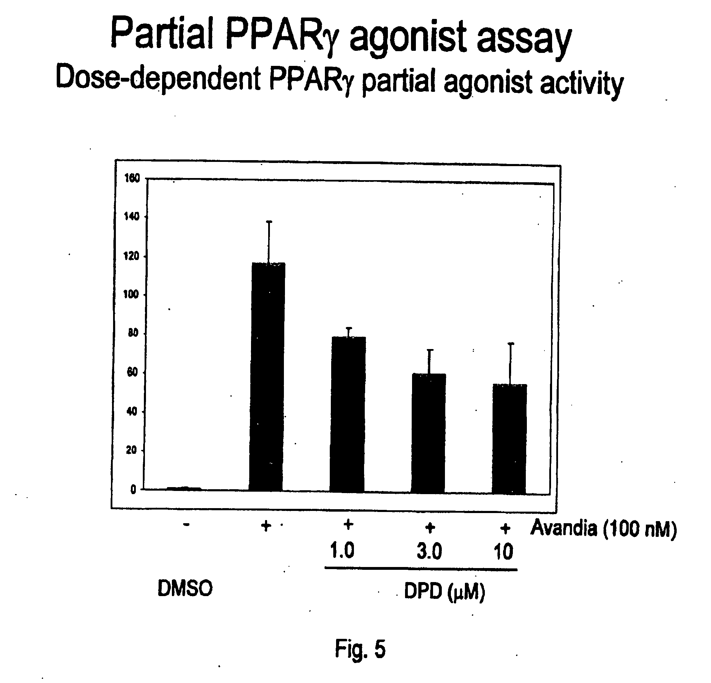

Briefly, transactivation assays are carried out essentially as described in Example 1 but 100nM Avandia (a full agonist) is added to each well, together with increasing concentrations of test compound (or no test compound as control). Compounds that reduce the transactivation by Avandia are PPAR partial agonists. The results are depicted in Fig 5.

PPAR gamma partial agonists are identified in this example by their ability to displace a known PPAR gamma agonist from binding to PPAR gamma, e.g., Avandina in this case. Alternatively, other known full agonists can be used, for example 300nM L165041 to identify partial agonists of PPAR delta using the transactivation assay essentially as described in Example 2.

### Example 27 Partial PPARgamma Agonist Ligand Displacement Assay

A further assay to identify partial PPARgamma agonists is performed using the Invitrogen POLARSCREEN PPAR competition assay, as follows for the analysis of binding to the PPARgamma ligand-binding pocket.
1. Dispense 20 microL 2X test compound in the cuvette
2. Add 20 microliter 2X PPAR-LBD/Fluormone Green Complex and mix
3. Incubate in the dark for 2 hours
4. Measure fluorescence polarization value
5. As control, use rosiglitazone (Avandia)

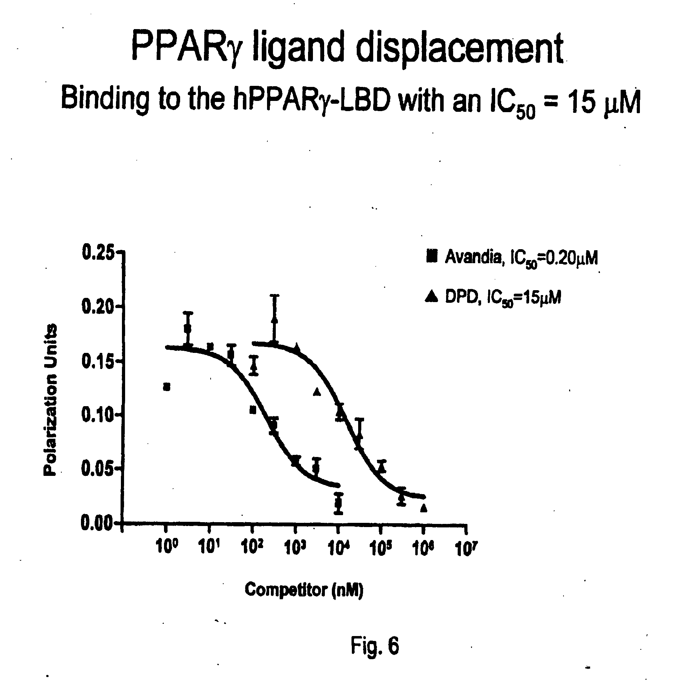

The results with DPD relative to AVANDIA are shown in Fig 6.

### Example 28 Glucose uptake assays

This example illustrates that compounds identified as PPAR gamma agonists also produce a physiological effect in cellular assays, expected of a PPAR gamma agonist, namely an effect on glucose uptake. Glucose uptake assays are important to establish the suitability of a compound for the treatment of insulin resistance.

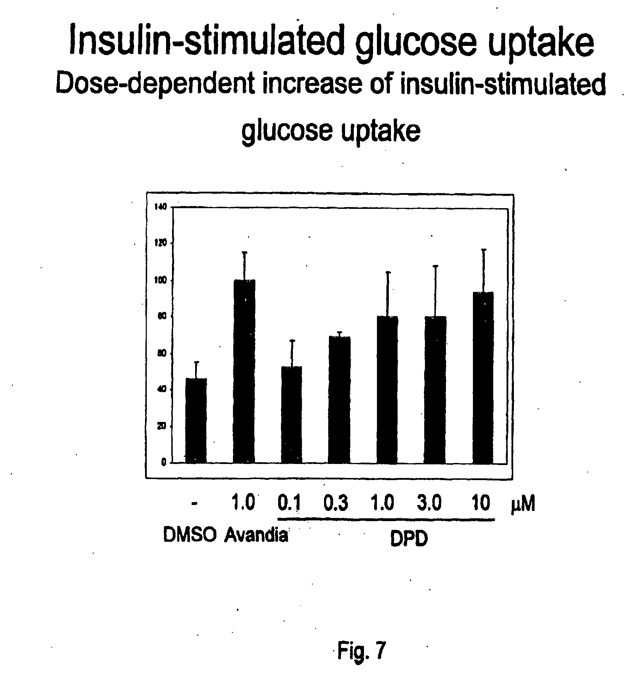

Briefly, 3T3-L1 preadipocytes are grown in 12-well plates until confluence. Cells are washed with serum-free DMEM and incubated with 1 ml of the same medium at 37°C for 1-2 h. The cells are then washed with Krebs-Ringer-Hepes (KRP) buffer and then incubated with 0.9 ml KRP buffer at 37°C for 30 min. Insulin is added to the cells at 0, 0.3, 1 and 3 nM final concentration and incubated for 15min at 37°C. Glucose uptake is initiated by the addition of 0.1 ml of Krebs-Ringer phosphate (KRP) buffersupplemented with 10mM [³H] 2-deoxy-D-glucose (1 mCi/l). After a 10 minute incubation at 37°C, the medium is aspirated and plates washed with ice-cold PBS to terminate the induced glucose uptake. The cells are lyzed with 0.5 ml 1% Triton X-100 and radioactivity levels determined using a scintilla-tion counter. The results are depicted in Figure 7.

In summary, DPD and a number of analogs referred to in Table II are demonstrated to be PPAR gamma agonists, whereas substance 10 appeared to be a PPAR gamma antagonist. Substances 7 and 13 are particularly interesting analogs as these show higher potency whilst causing low or no adipocyte differentiation.

**Table II**

| **SN** | **Structure** | **PPAR** | **Adip.** |
|---|---|---|---|
| 1 | 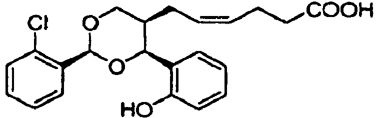 | P | 0 |
| 2 | 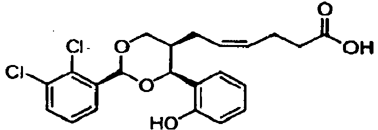 | P | 0 |
| 3 | 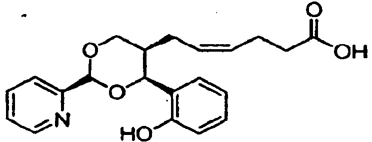 | - | - |
| 4 | 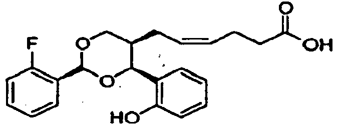 | P+ | 0 |
| 5 | 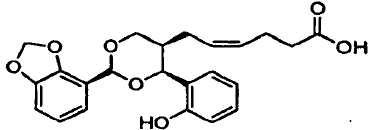 | P- | 0 |
| 6 | 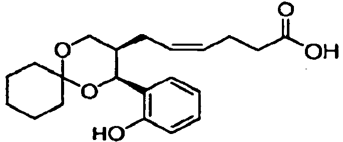 | P | 0 |
| 7 | 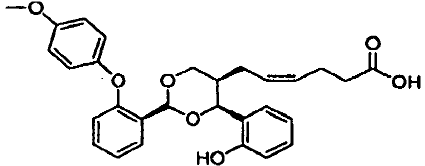 | P+++ | 0 |
| 8 | 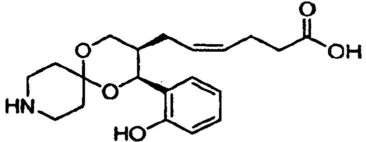 | Boc derivative | -1 |
| | | Inactive | |
| 9 | 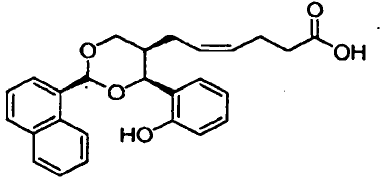 | P | 0 |
| 10 | 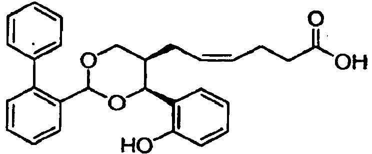 | Antagonist | 0 |
| 11 | 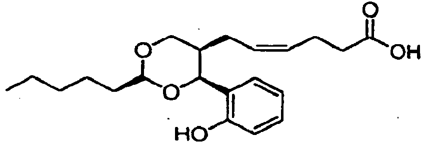 | P+ | +1 |
| 12 | 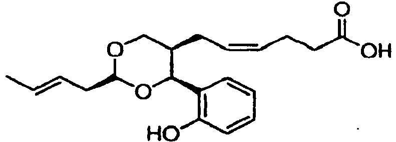 | P | -1 |
| 13 | 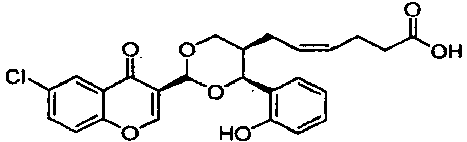 | P+++ | -1 |
| 14 | 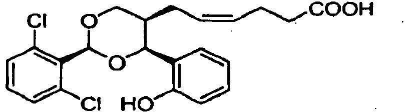 | | |
| 15 | 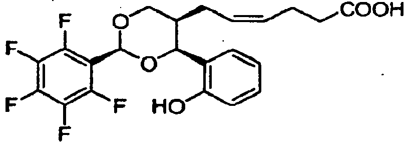 | | |
| 16 | 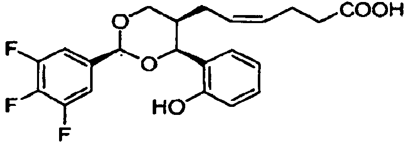 | | |
| 17 | 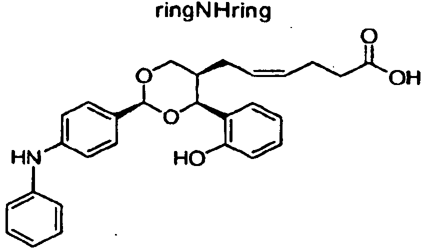 | | |
| 18 | 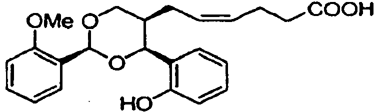 | P | -1 |
| 19 | 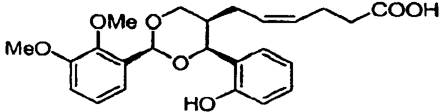 | P | 0 |
| 20 | 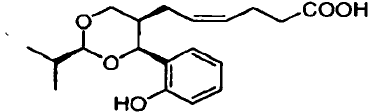 | P | 0 |
| 21 | 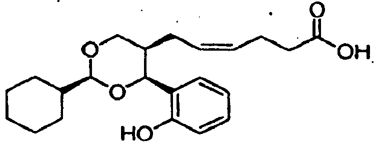 | P+ | 0 |
| 22 | 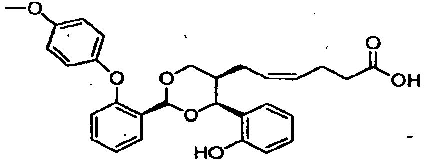 | | |
| 23 | 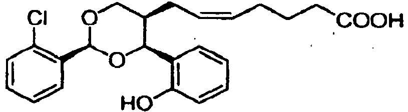 | P+ | +1 |
| 24 | 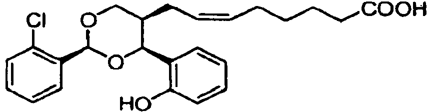 | P+ | - |
| 25 | 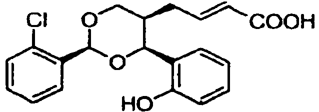 | - | - |
| 26 | 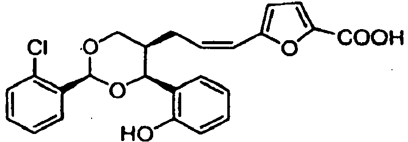 | | |
| 27 | 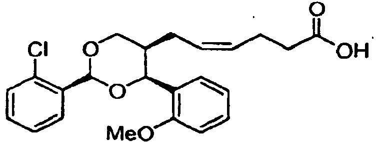 | Inactive | - |
| 28 | 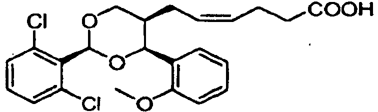 | Inactive | - |
| 29 | 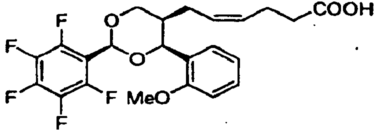 | P | - |
| 30 | 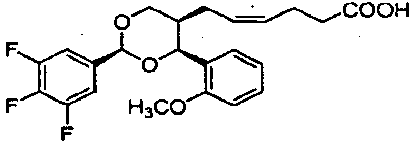 | P | - |
| 31 | 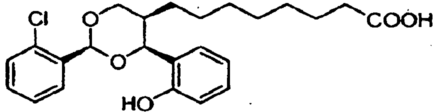 | P+ | 0 |
| 32 | 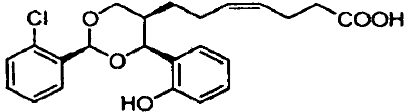 | P++ | - |
| 33 | 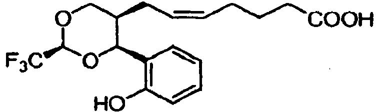 | P | - |
| 34 | 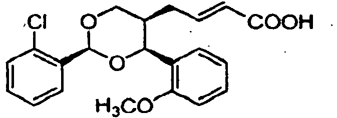 | P | - |
| 35 | 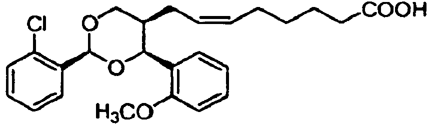 | P | - |
| 36 | 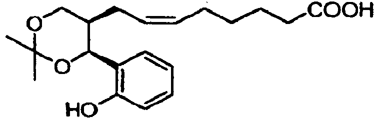 | Inactive | - |
| 37 | 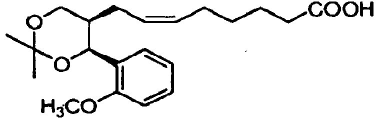 | Inactive | 0 |
| 38 | 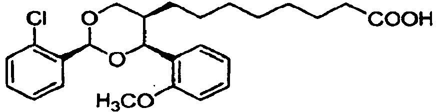 | P+ | - |
| 39 | 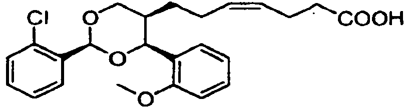 | - | - |

### Example 29 Thromboxane Receptor activity

This example demonstrates that although both enantiomers of substance 1 have PPAR agonist activity, only one acts as a thromboxane receptor antagonist. Each enantiomer of DPD (SN1 in Table II) was isolated by chiral chromatography under the following conditions:
Column: 250x4.6 mm Chiralpak AD-H 5 □m
   Mobile phase: 80/20/0.1 n-Heptane/Ethanol/Trifluoroacetic acid
   Flow rate: 1 ml/min
   Detection: UV at 230 nm
   Temperature: 25 C
   Samples were dissolved in 80/20 n-Heptane/Ethanol
Enantiomer 1 elutes first on the chiral column and Enantiomer 2 eluted second on the chiral column.

The enantiomers 1 and 2 were tested on radioligand binding assays for thromboxane receptor binding essentially as described by Hedberg et al. (1988) J Pharmacol. Exp. Ther. 245:786-792 and Saussy et al. (1986) J. Biol. Chem. 261: 3025-3029. Whereas Enantiomer 1 was found to be a potent thromboxane receptor binder (IC50 0.841nM) enantiomer 2 did not appear to bind (IC50 >10nM).

It might be desirable to treat certain patient populations with an enantiomer that acts as a thromboxane receptor antagonist, in which case enantiomer 1 would be administered (for example, where the cardiovascular benefits of thromboxane receptor antagonists would be desired simultaneously). Where it would be desirable not to have an effect on thromboxane receptor (eg where no additional cardiovascular effects are needed, such as when a patient needs no such treatment or when a patient is already receiving different treatment), enantiomer 2 would be administered beneficially.

### Example 30 Inhibition of Cancer Cell Proliferation

This example demonstrates the anti-profiferative effect of DPD on the growth of cancer cells and illustrates the utility of the analogs described herein for the treatment of cancer.

A human cervical carcinoma cell line (HeLa) engineered to stably express a recombinant biosensor reporter activity and a non-cancer cell line, HaCaT (Boukamp et al., 1988, J. Cell Biol 106(3):761-771) was used with a Caspa Tag kit (commercially available eg from Chemicon). The detection of proliferation was carried out using a high content screening automated flow cytometer.

DPD (Substance 1) was diluted to 20microM in cell culture medium without serum adjusted to 1% DMSO. Detection of cellular events was performed in duplicate in 96-well plates after 48 hours of treatment in the FL2 (dilution of a proliferative marker) channels of a HTS flow cytometer (FACS Calibur HTS, Becton Dickenson). At day 1, cells were seeded in 96 well plates. Cells were treated with DPD and the appropriate controls on day 2 and analysis was carried out on day 4.

DPD inhibited 90% proliferation of HeLa cells at 20microM. DPD had no effect on the non-cancer cell line HaCaT, thus establishing a selective antiproliferative effect on cancer cells by DPD (SN1 in Table II).

## Claims

1. A 1,3-dioxane derivative or a pharmaceutically acceptable salt thereof for use in treating a peroxisome-proliferator activated receptor (PPAR) responsive disease or condition selected from insulin resistance, diabetes, metabolic syndrome, obesity, pre-diabetes, diabetes, dyslipidemia, and wound healing, wherein the derivative is represented by the formula: 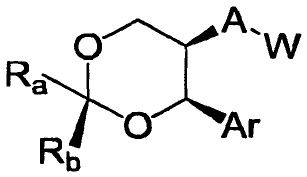 wherein
A is a branched or linear carbon chain of 3 to 7 carbons with up to 2 double bonds;
W is COOH, OH, NH₂, SO₃H, OSO₃H, or an aromatic group selected from the group consisting of phenyl, 1- or 2-naphthyl, pyridine, furan, 2-methylpyridine and a dioxolane, each optionally substituted with COOH, OH or NaH₂;
Ar is a phenyl, 1-naphthyl, 2-naphthyl, or a 5 or 6 membered heterocyclic aromatic group optionally substituted with hydoxy or methoxy in the ortho, meta and/or para positions;
and,
Ra and Rb are independently hydrogen, 2-6C alkenyl, 1-8C alkyl optionally with up to three halogeno substiuents, pentafluorophenyl, aryl or aryl(1-4C)allcyl, said aryl or aryl(1-4C) alkyl substituents being optionally substituted with up to five substituents selected from halogeno, (1-6C)allcyl, branched or linear (1-6C)alkoxy, (1-4C)alkylenedioxy, trifluoromethyl, cyano, nitro, hydroxyl, (2-6C)alkanoyloxy, (1-6C)alkylthio, (1-6C)alkanesulphonyl, (1-6C)alkanoylamino and oxapolymethylene of 2 to 4 carbon atoms, or Ra and Rb together form polymethylene of 2 to 7 carbon atoms, optionally having one or two (1-4C)alkyl substituents.

2. The derivative for use in a method for the treatment of a disease or condition according to claim 1, wherein A is a linear carbon chain of 3 to 7 carbons, and W is COOH.

3. The derivative for use in a method for the treatment of a disease or condition according to claim 2, wherein said carbon chain includes a double bond between C2-C3 or C3-C4.

4. The derivative for use in a method for the treatment of a disease or condition according to any one of the preceding claims, wherein Ar is a 2-OH or 2-OMe substituted phenyl or naphthyl group.

5. The derivative for use in a method for the treatment of a disease or condition according to any one of the preceding claims, wherein Ra is H and Rb is an aryl group selected from the group consisting of phenyl, benzyl, 2- or 3- or 4-pyridine, furan, biphenyl 1-naphthyl and 2-naphthyl, each optionally substituted with halogen, OH, O-alkyl, amino, N-monoalkyl, N-dialkyl, nitroalkyl or thioalkyl.

6. The derivative for use in a method for the treatment of a disease or condition according to claim 1, wherein said compound is a 2,4-diphenyl-1,3-dioxane derivative of Formula II or a pharmaceutically acceptable salt thereof, wherein formula II is represented by: 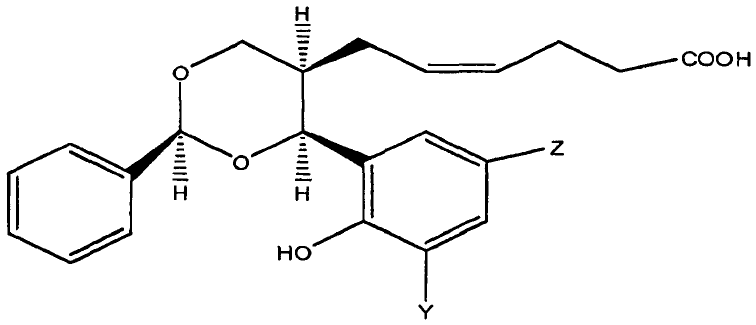 wherein X is selected from fluoro, chloro, bromo, trifluoromethyl, optionally substituted phenyl, cyano, methoxy and nitro, or the phenyl-X group can be an optionally substituted chromen derivative; and Y and Z are individually hydrogen or halogeno.

7. The derivative for use in a method for the treatment of a disease or condition according to claim 6, wherein the groups at positions 2, 4 and 5 of the dioxane ring in the derivative represented by formula II have cis-relative stereochemistry.

8. The derivative for use in a method for the treatment of a disease or condition according to claim 6 or 7, wherein X is selected from 2-fluoro, 2-chloro, 2-bromo, 2-cyano, 2-trifluoromethyl, 3-fluoro, 3-chloro, 3-cyano, 3-nitro, 3-methoxy, 4-chloro, 4-cyano, 4-nitro and 4-methoxy; Y is hydrogen or fluoro; and Z is hydrogen.

9. The derivative for use in a method for the treatment of a disease or condition according to claim 8, wherein X is selected from 2-chloro, 3-chloro, 2-cyano, 4-cyano, 3-nitro and 4-nitro; and Y and Z are hydrogen.

10. The derivative for use in a method for the treatment of a disease or condition according to claim 8, wherein said derivative is 4(Z)-6-(2-o-chlorophenyl-4-o-hydroxyphenyl-1,3-dioxan-cis-5-yl)hexenoic acid or a pharmaceutically acceptable salt thereof.

11. The derivative for use in a method for the treatment of a disease or condition according to claim 1, wherein
A is a branched or linear carbon chain of 3 to 7 carbons with up to 2 double bonds;
W is COOH, OH, NH₂, SO₃H, OSO₃H, or an aromatic group selected from the group consisting of phenyl, 1- or 2-naphthyl, pyridine, furan, 2-methylpyridine and a dioxolane, each optionally substituted with COOH, OH or NH₂
Ar is a phenyl, 1-naphthyl, 2-naphthyl, biphenyl (4-methoxyphenoxy)-phenyl, or a 5 or 6 membered heterocyclic aromatic group selected from substituted or unsubstituted 2-pyridine, 3-pyridine, thiophene, and furan,
and,
Ra is H and Rb is an aryl group or a heterocycle optionally substituted with three different substituents selected from the group consisting of halogen, OH, O-alkyl, O-aryl, amino or N-monoalkyl or N-dialkyl or N-monoaryl or N-diaryl, nitro, thioalkyl or oxo.

12. The derivative for use in a method for the treatment of a disease or condition according to claim 11, wherein A is a five carbon linear chain with one double bond, W is COOH, Ar is phenyl substituted in the o-position by OH or OMe and Rb is a heterocycle or a phenyl group substituted with O-aryl.

13. The derivative for use in a method for the treatment of a disease or condition according to claim 11, wherein said derivative is 4(Z)-6-(2-[4-methoxyphenoxy-o-phenyl]-4-o-hydroxyphenyl-1,3-dioxan-cis-5-yl)hexenoic acid or a pharmaceutically acceptable salt thereof; or said derivative is 4(Z)-6-(2-3-[6-chloro-4H-chromen-4-one ]-4-o-hydroxyphenyl-1,3-dioxan-cis-5-yl)hexenoic acid or a pharmaceutically acceptable salt thereof.

14. The derivative for use in a method for the treatment of a disease or condition according to any preceding claim, wherein said compound is present as a pharmaceutically acceptable salt selected from alkali metal and alkaline earth metal salts, aluminium and ammonium salts, and from salts with organic amines and quaternary bases forming physiologically acceptable cations.

15. A derivative for use in a method for the treatment of a disease or condition according to claim 1, wherein the derivative is selected from: 4(Z)-6-(2-[4-methoxyphenoxy-o-phenyl]-4-o-hydroxyphenyl-1,3-dioxan-cis-5-yl)hexenoic acid; 4(Z)-6-(2-[6-chloro-4H-chromen-4-one]-4-o-hydroxyphenyl-1,3-dioxan-cis-5-yl)hexenoic acid; (Z)-6-((2S,4S,5R)-2-(2-chlorophenyl)-4-(2-hydroxyphenyl)-1,3-dioxan-5-yl)hex-4-enoic acid; (Z)-6-((2S,4S,5R)-2-(2,6-dichlorophenyl)-4-(2-methoxyphenyl)-1,3-dioxan-5-yl)hex-4-enoic acid; (Z)-6-((2S,4S,5R)-4-(2-methoxyphenyl)-2-(perfluorophenyl)-1,3-dioxan-5-yl)hex-4-enoic acid; (E)-4-((2S,4S,5R)-2-(2-chlorophenyl)-4-(2-methoxyphenyl)-1,3-dioxan-5-yl)but-2-enoic acid; (Z)-8-((2S,4S,5R)-2-(2-chlorophenyl)-4-(2-methoxyphenyt)-1,3-dioxan-5-yl)oct-6-enoic acid; (Z)-8-((4S,5R)-4-(2-methoxyphenyl)-2,2-dimethyl-1,3-dioxan-5-yt)oct-6-enoic acid; 8-((2S,4S,5R)-2-(2-chlorophenyl)-4-(2-methoxyphenyl)-1,3-dioxan-5-yl)octanoic acid; and (Z)-7-((2S)4S,5R)-2-(2-chlorophenyl)-4-(2-methoxyphenyl)-1,3-dioxan-5-yl)hept-4-enoic acid.

## Patentansprüche

1. 1,3-Dioxanderivat oder pharmazeutisch akzeptables Salz davon, zum Gebrauch bei der Behandlung einer Krankheit bzw. eines Leidens, das auf Peroxisom-Proliferator-aktivierte Rezeptoren (PPAR) anspricht, ausgewählt aus Insulinresistenz, Diabetes mellitus, metabolischem Syndrom, Adiposität, Prädiabetes, Diabetes mellitus, Dyslipidemie und Wundheilung, wobei das Derivat durch folgende Formal dargestellt wird: 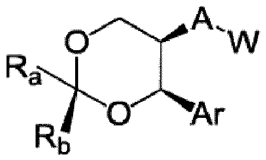 wobei
A eine verzweigte oder lineare Kohlenstoffkette mit 3 bis 7 Kohlenstoffen mit bis zu 2 Doppelbindungen darstellt;
W COOH, OH, NH₂, SO₃H, OSO₃H oder eine aromatische Gruppe ist, ausgewählt aus der Gruppe, die aus Phenyl, 1- oder 2-Naphthyl, Pyridin, Furan, 2-Methylpyridin und einem Dioxolan besteht, jeweils wahlweise mit COOH, OH oder NH₂ substituiert;
Ar ein Phenyl, 1-Naphthyl, 2-Naphthyl oder eine 5- oder 6-gliedrige heterozyklische aromatische Gruppe ist, wahlweise mit Hydroxy oder Methoxy an den ortho-, meta- und/oder para-Stellen substitutiert;
und
Ra und Rb, unabhängig von einander, Wasserstoff, 2-6C Alkenyl, 1-8C Alkyl, wahlweise mit bis zu drei Halogen-Substituenten, Pentafluorphenyl, Aryl oder Aryl-(1-4C)-Alkyl, besagte Aryl- oder Aryl-(1-4C)-Alkyl-Substituenten wahlweise mit bis zu fünf Substituenten substituiert, die ausgewählt sind aus Halogeno, (1-6C)-Alkyl, verzweigtem oder linearem (1-6C)-Alkoxy, (1-4C)-Alkylendioxy, Trifluormethyl, Cyano, Nitro, Hydroxyl, (2-6C)-Alkanoyloxy, (1-6C)-Alkylthio, (1-6C)-Alkansulphonyl, (1-6C)-Alkanoylamino und Oxapolymethylen mit 2 bis 4 Kohlenstoffatomen sind, oder Ra und Rb zusammenPolymethylen mit 2 bis 7 Kohlenstoffatomen bilden, wahlweise mit einem oder zwei (1-4C)-Alkyl-Substituenten.

2. Derivat für den Gebrauch in einem Verfahren zur Behandlung einer Krankheit oder eines Leidens gemäß Anspruch 1, wobei A eine lineare Kohlenstoffkette mit 3 bis 7 Kohlenstoffatomen ist und W COOH ist.

3. Derivat für den Gebrauch in einem Verfahren zur Behandlung einer Krankheit oder eines Leidens gemäß Anspruch 2, wobei die besagte Kohlenstoffkette eine Doppelbindung zwischen C2-C3 oder C3-C4 umfasst.

4. Derivat für den Gebrauch in einem Verfahren zur Behandlung einer Krankheit oder eines Leidens gemäß irgendeinem der vorhergehenden Ansprüche, wobei Ar eine 2-OH- oder 2-OMe-substituierte Phenyl- oder Naphthylgruppe ist.

5. Derivat für den Gebrauch in einem Verfahren zur Behandlung einer Krankheit oder eines Leidens gemäß irgendeinem der vorhergehenden Ansprüche, wobei Ra H ist und Rb eine Arylgruppe ist, die aus der Gruppe ausgewählt ist, die aus Phenyl, Benzyl, 2- oder 3- oder 4-Pyridin, Furan, Biphenyl, 1-Naphthyl und 2-Naphthyl besteht, jeweils wahlweise substituiert mit Halogen, OH, O-Alkyl, Amino, N-Monoalkyl, N-Dialkyl, Nitroalkyl oder Thioalkyl.

6. Derivat für den Gebrauch in einem Verfahren zur Behandlung einer Krankheit oder eines Leidens gemäß Anspruch 1, wobei die besagte Verbindung ein 2,4-Diphenyl-1,3-dioxan-Derivat der Formel II oder ein pharmazeutisch akzeptables Salz davon ist, wobei die Formel II dargestellt wird von: 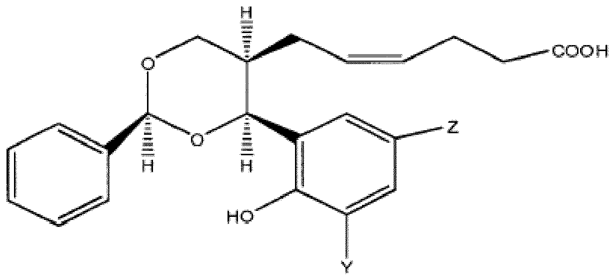 wobei X ausgewählt ist aus Fluor-, Chlor-, Brom-, Trifluormethyl, wahlweise substituiertem Phenyl, Cyano, Methoxy und Nitro, oder die Phenyl-X-Gruppe ein wahlweise substituiertes Chromenderivat sein kann, und Y und Z einzeln Wasserstoff oder Halogeno sind.

7. Derivat für den Gebrauch in einem Verfahren zur Behandlung einer Krankheit oder eines Leidens gemäß Anspruch 6, wobei die Gruppen an den Stellen 2, 4 und 5 des Dioxanringes in dem Derivat, das durch Formel II dargestellt wird, cis-relative Stereochemie besitzen.

8. Derivat für den Gebrauch in einem Verfahren zur Behandlung einer Krankheit oder eines Leidens gemäß Anspruch 6 oder 7, wobei X ausgewählt ist aus 2-Fluor-, 2-Chlor-, 2-Brom-, 2-Cyano-, 2-Trifluormethyl, 3-Fluoro, 3-Chloro, 3-Cyano, 3-Nitro, 3-Methoxy, 4-Chloro, 4-Cyano, 4-Nitro und 4-Methoxy; Y Wasserstoff oder Fluoro ist; und Z Wasserstoff ist.

9. Derivat für den Gebrauch in einem Verfahren zur Behandlung einer Krankheit oder eines Leidens gemäß Anspruch 8, wobei X ausgewählt ist aus 2-Chloro, 3-Chloro, 2-Cyano, 4-Cyano, 3-Nitro und 4-Nitro; und Y und Z Wasserstoff sind.

10. Derivat für den Gebrauch in einem Verfahren zur Behandlung einer Krankheit oder eines Leidens gemäß Anspruch 8, wobei das besagte Derivat 4(Z)-6-(2-o-Chlorphenyl-4-o-hydroxyphenyl-1,3-dioxan-cis-5-yl)-Hexensäure oder ein pharmazeutisch akzeptables Salz davon ist.

11. Derivat für den Gebrauch in einem Verfahren zur Behandlung einer Krankheit oder eines Leidens gemäß Anspruch 1, wobei
A eine verzweigte oder lineare Kohlenstoffkette mit 3 bis 7 Kohlenstoffatomen mit bis zu 2 Doppelbindungen darstellt;
W COOH, OH, NH₂, SO₃H, OSO₃H oder eine aromatische Gruppe ist, ausgewählt aus der Gruppe, die aus Phenyl, 1- oder 2-Naphthyl, Pyridin, Furan, 2-Methylpyridin und einem Dioxolan besteht, jeweils wahlweise mit COOH, OH oder NH₂ substituiert;
Ar ein Phenyl, 1-Naphthyl, 2-Naphthyl, Biphenyl-(4-methoxyphenoxy)-phenyl oder eine 5- oder 6-gliedrige heterozyklische aromatische Gruppe ist, ausgewählt aus substituiertem oder nicht substituiertem 2-Pyridin, 3-Pyridin, Thiophen und Furan;
und
Ra H ist und Rb eine Arylgruppe ist oder eine heterocyclische Verbindung, wahlweise substituiert mit drei verschiedenen Substituenten, ausgewählt aus der Gruppe, die aus Halogen, OH, O-Alkyl, O-Aryl, Amino oder N-Monoalkyl oder N-Dialkyl oder N-Monoaryl oder N-Diaryl, Nitro, Thioalkyl oder Oxo besteht.

12. Derivat für den Gebrauch in einem Verfahren zur Behandlung einer Krankheit oder eines Leidens gemäß Anspruch 11, wobei A eine lineare Kette mit 5 Kohlenstoffatomen und einer Doppelbindung ist, W COOH ist, Ar in der o-Stellung mit OH oder OMe phenylsubstituiert ist und Rb eine heterocyclische Verbindung oder eine mit O-Aryl substituierte Phenylgruppe ist.

13. Derivat für den Gebrauch in einem Verfahren zur Behandlung einer Krankheit oder eines Leidens gemäß Anspruch 11, wobei das besagte Derivat 4(Z)-6-(2-[4-Methoxy-phenoxy-o-phenyl]-4-o-hydroxyphenyl-1,3-dioxan-cis-5-yl)-Hexensäure oder ein pharmazeutisch akzeptables Salz davon ist; oder das besagte Derivat 4(Z)-6-(2-3-[6-Chlor-4H-chromen-4-on]-4-o-hydroxyphenyl-1,3-dioxan-cis-5-yl)-Hexensäure oder ein pharmazeutisch akzeptables Salz davon ist.

14. Derivat für den Gebrauch in einem Verfahren zur Behandlung einer Krankheit oder eines Leidens gemäß irgendeinem der vorhergehenden Ansprüche, wobei die besagte Verbindung vorhanden ist als pharmazeutisch akzeptables Salz, ausgewählt aus Alkalimetall- und Erdalkalimetallsalzen, Aluminum- und Ammoniumsalzen, und aus Salzen mit organischen Aminen und quaternären Basen, die physiologisch akzeptable Kationen bilden.

15. Derivat für den Gebrauch in einem Verfahren zur Behandlung einer Krankheit oder eines Leidens gemäß Anspruch 1, wobei das Derivat ausgewählt ist aus: 4(Z)-6-(2-[4-Methoxyphenoxy-o-phenyl]-4-o-hydroxyphenyl-1,3-dioxan-cis-5-yl)-Hexensäure; 4(Z)-6-(2-[6-Chlor-4H-chromen-4-on]-4-o-hydroxyphenyl-1,3-dioxan-cis-5-yl)-Hexensäure; (Z)-6-((2S,4S,SR)-2-(2-Chlorphenyl)-4-(2-hydroxyphenyl(1-3-dioxan-5-yl)-Hex-4-ensäure; (Z)-6-((2S,4S,SR)-2-(2,6-Dichlorphenyl)-4-(2-methoxyphenyl)-1,3-dioxan-5-yl)-Hex-4-ensäure; (Z)-6-((2S,4S,SR)-4-(2-Methoxyphenyl)-2-(perfluorphenyl)-1,3-dioxan-5-yl)-Hex-4-ensäure; (E)-4-((2S,4S,SR)-2-(2-Chlorphenyl)-4-(2-methoxyphenyl)-1,3-dioxan-5-yl)-But-2-ensäure; (Z)-8-((2S,4S,SR)-2-(2-Chlorphenyl)-4-(2-methoxyphenyl)-1,3-dioxan-5-yl)-Okt-6-ensäure; (Z)-8-((4S,5R)-4-(2-Methoxyphenyl)-2.2-dimethyl-1,3-dioxan-5-yl)-Okt-6-ensäure; 8-((2S,4S,5R)-2-(2-Chlorophenyl)-4-(2-methoxyphenyl)-1,3-dioxan-5-yl)-Oktensäure; und (Z)-7-((2S,4S,5R)-2-(2-Chlorophenyl)-4-(2-methoxyphenyl)1,3-dioxan-5-yl)-Hept-4-ensäure.

## Revendications

1. Dérivé de 1,3-dioxane ou sel pharmaceutiquement acceptable de celui-ci destiné à être utilisé dans le traitement d'une maladie ou d'un état sensible au récepteur activé par les proliférateurs de peroxysomes (PPAR) choisi parmi la résistance à l'insuline, le diabète, le syndrome métabolique, l'obésité, le pré-diabète, le diabète, la dyslipidémie et la cicatrisation des plaies, le dérivé étant représenté par la formule : 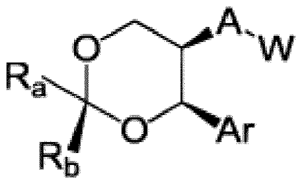 dans laquelle .
- A représente une chaîne carbonée ramifiée ou linéaire de 3 à 7 carbones avec jusqu'à 2 doubles liaisons ;
- W représente COOH, OH, NH₂, SO₃H, OSO₃H ou un groupe aromatique choisi dans le groupe consistant en phényle, 1- ou 2-naphthyle, pyridine, furane, 2-méthylpyridine et un dioxolane, chacun étant facultativement substitué par COOH, OH ou NH₂ ;
- Ar représente phényle, 1-naphtyle, 2-naphtyle, ou un groupe aromatique hétérocyclique à 5 ou 6 chaînons facultativement substitué par hydroxy ou méthoxy dans les positions ortho, méta et/ou para ; et,
- Ra et Rb représentent indépendamment hydrogène, alcényle en C₂-C₆, alkyle en C₁-C₈ facultativement avec jusqu'à trois substituants halogéno, pentafluorophényle, aryle ou aryl-alkyle en C₁-C₄, lesdits substituants aryle ou aryl-alkyle en C₁-C₄ étant facultativement substitués par jusqu'à cinq substituants choisis parmi halogéno, alkyle en C₁-C₆, alcoxy en C₁-C₆ ramifié ou linéaire, alkylène dioxy en C₁-C₄, trifluorométhyle, cyano, nitro, hydroxyle, alcanoyloxy en C₂-C₆, alkylthio en C₁-C₆, alcanesulfonyle en C₁-C₆, alcanoylamino en C₁-C₆ et oxapolyméthylène de 2 à 4 atomes de carbone ; ou
- Ra et Rb forment ensemble un polyméthylène de 2 à 7 atomes de carbone, ayant facultativement un ou deux substituants alkyle en C₁-C₄.

2. Dérivé destiné à être utilisé dans un procédé pour le traitement d'une maladie ou d'un état, selon la revendication 1, dans lequel A représente une chaîne carbonée linéaire de 3 à 7 carbones, et W représente COOH.

3. Dérivé destiné à être utilisé dans un procédé pour le traitement d'une maladie ou d'un état, selon la revendication 2, dans lequel ladite chaîne carbonée comprend une double liaison entre C₂-C₃ ou C₃-C₄.

4. Dérivé destiné à être utilisé dans un procédé pour le traitement d'une maladie ou d'un état, selon l'une quelconque des revendications précédentes, dans lequel Ar représente un groupe phényle ou naphtyle substitué par 2-OH ou 2-OMe.

5. Dérivé destiné à être utilisé dans un procédé pour le traitement d'une maladie ou d'un état, selon l'une quelconque des revendications précédentes, dans lequel Ra représente H et Rb représente un groupe aryle choisi dans le groupe consistant en phényle, benzyle, 2- ou 3- ou 4-pyridine, furane, biphenyle, 1-naphtyle et 2-naphtyle, chacun étant facultativement substitué par halogène, OH, 0-alkyle, amino, N-monoalkyle, N-dialkyle, nitroalkyle ou thioalkyle.

6. Dérivé destiné à être utilisé dans un procédé pour le traitement d'une maladie ou d'un état, selon la revendication 1, dans lequel ledit composé est un dérivé 2,4-diphényl-1,3-dioxane de Formule II ou un sel pharmaceutiquement acceptable de celui-ci, dans lequel la Formule II est représentée par : 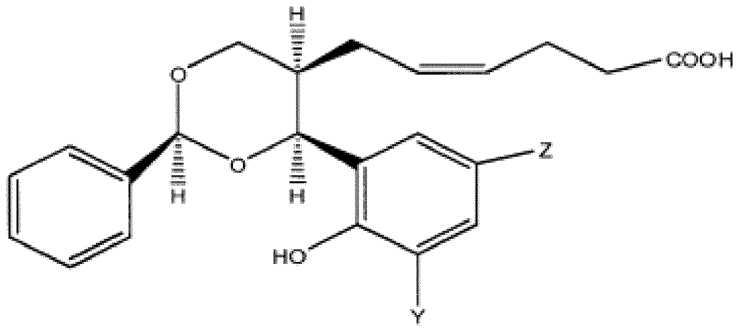 dans lequel X est choisi parmi fluoro, chloro, bromo, trifluorométhyle, phényle facultativement substitué, cyano, méthoxy et nitro, ou le groupe phényl-X peut être un dérivé de chromène facultativement substitué ; et Y et Z représentent individuellement hydrogène ou halogéno.

7. Dérivé destiné à être utilisé dans un procédé pour le traitement d'une maladie ou d'un état, selon la revendication 6, dans lequel les groupes dans les positions 2, 4 et 5 du noyau dioxane dans le dérivé représenté par la formule II ont une stéréochimie relative cis.

8. Dérivé destiné à être utilisé dans un procédé pour le traitement d'une maladie ou d'un état, selon l'une des revendications 6 ou 7, dans lequel X est choisi parmi 2-fluoro, 2-chloro, 2-bromo, 2-cyano, 2-trifluorométhyle, 3-fluoro, 3-chloro, 3-cyano, 3-nitro, 3-méthoxy, 4-chloro, 4-cyano, 4-nitro et 4-méthoxy ; Y représente hydrogène ou fluoro ; et Z représente hydrogène.

9. Dérivé destiné à être utilisé dans un procédé pour le traitement d'une maladie ou d'un état, selon la revendication 8, dans lequel X est choisi parmi 2-chloro, 3-chloro, 2-cyano, 4-cyano, 3-nitro et 4-nitro ; et Y et Z représentent hydrogène.

10. Dérivé destiné à être utilisé dans un procédé pour le traitement d'une maladie ou d'un état, selon la revendication 8, dans lequel ledit dérivé est l'acide 4(2)-6-(2-o-chlorophényl-4-o-hydroxyphényl-1,3-dioxan-cis-5-yl)hexénoïque ou un sel pharmaceutiquement acceptable de celui-ci.

11. Dérivé destiné à être utilisé dans un procédé pour le traitement d'une maladie ou d'un état, selon la revendication 1, dans lequel
- A représente une chaîne carbonée ramifiée ou linéaire de 3 à 7 atomes de carbone avec jusqu'à 2 doubles liaisons ;
- W représente COOH, OH, NH₂, SO₃H, OSO₃H, ou un groupe aromatique choisi dans le groupe consistant en phényle, 1- ou 2-naphtyle, pyridine, furane, 2-méthylpyridine et un dioxolane, chacun étant facultativement substitué par COOH, OH ou NH₂ ;
- Ar représente un phényle, 1-naphtyle, 2-naphtyle, biphényl(4-méthoxyphénoxy)-phényle, ou un groupe aromatique hétérocyclique à 5 ou 6 chaînons choisi parmi les 2-pyridine, 3-pyridine, thiophène et furane substitués ou non substitués ; et
- Ra représente H et Rb représente un groupe aryle ou un hétérocycle facultativement substitué par trois substituants différents choisi dans le groupe consistant en halogène, OH, O-alkyle, O-aryle, amino ou N-monoalkyle ou N-dialkyle ou N-monoaryle ou N-diaryle, nitro, thioalkyle ou oxo.

12. Dérivé destiné à être utilisé dans un procédé pour le traitement d'une maladie ou d'un état, selon la revendication 11, dans lequel A est une chaîne linéaire à cinq carbones avec une double liaison, W représente COOH, Ar représente phényle substitué en position o par OH ou OMe et Rb est un hétérocycle ou un groupe phényle substitué par O-aryle.

13. Dérivé destiné à être utilisé dans un procédé pour le traitement d'une maladie ou d'un état, selon la revendication 11, dans lequel ledit dérivé est l'acide 4(Z)-6-(2-[4-méthoxyphénoxy-o-phényl]-4-o-hydroxyphényl-1,3-dioxan-cis-5-yl)hexénoïque ou un sel pharmaceutiquement acceptable de celui-ci ; ou ledit dérivé est l'acide 4(Z)-6-(2-3-[6-chloro-4H-chromén-4-one]-4-o-hydroxyphényl-1,3-dioxan-cis-5-yl)hexénoïque ou un sel pharmaceutiquement acceptable de celui-ci.

14. Dérivé destiné à être utilisé dans un procédé pour le traitement d'une maladie ou d'un état, selon l'une quelconque des revendications précédentes, dans lequel ledit composé est présent en tant que sel pharmaceutiquement acceptable choisi parmi des sels de métaux alcalins et de métaux alcalino-terreux, des sels d'aluminium et d'ammonium, et des sels avec des amines organiques et des bases quaternaires formant des cations physiologiquement acceptables.

15. Dérivé destiné à être utilisé dans un procédé pour le traitement d'une maladie ou d'un état, selon la revendication 1, dans lequel le dérivé est choisi parmi : l'acide 4(Z)-6-(2-[4-méthoxyphénoxy-o-phényl]-4-o-hydroxyphényl-1,3-dioxan-cis-5-yl)hexénoïque ; l'acide 4(Z)-6-(2-[6-chloro-4H-chromén-4-one]-4-o-hydroxyphényl-1,3-dioxan-cis-5-yl)hexénoïque ; l'acide (Z)-6-((2S,4S,5R)-2-(2-chlorophényl)-4-(2-hydroxyphényl)-1,3-dioxan-5-yl)hex-4-énoïque ; l'acide (Z)-6-((2S,4S,5R)-2-(2,6-dichlorophényl)-4-(2-méthoxyphényl)-1,3-dioxan-5-yl)hex-4-énoïque ; l'acide (Z)-6-((2S,4S,5R)-4-(2-méthoxyphényl)-2-(perfluorophényl)-1,3-dioxan-5-yl)hex-4-énoïque ; l'acide (E)-4-((2S,4S,5R)-2-(2-chlorophényl)-4-(2-méthoxyphényl)-1,3-dioxan-5-yl)but-2-énoïque ; l'acide (Z)-8-((2S,4S,5R)-2-(2-chlorophényl)-4-(2-méthoxyphényl)-1,3-dioxan-5-yl)oct-6-énoïque ; l'acide (Z)-8-((4S,5R)-4-(2-méthoxyphényl)-2,2-diméthyl-1,3-dioxan-5-yl)oct-6-énoïque ; l'acide 8-((2S,4S,5R)-2-(2-chlorophényl)-4-(2-méthoxyphényl)-1,3-dioxan-5-yl)octanoïque ; et l'acide (Z)-7-((2S,4S,5R)-2-(2-chlorophényl)-4-(2-méthoxyphényl)-1,3-dioxan-5-yl)hept-4-énoïque.
